# EUROPEAN PATENT SPECIFICATION

(11) **EP 3 102 184 B1**
(45) Date of publication and mention of the grant of the patent: **30.01.2019**
(21) Application number: 15706976.6
(22) Date of filing: 06.02.2015
(51) Int. Cl.: A61K 9/08, A61K 9/19, A61K 47/02, A61K 47/20, A61K 38/26, A61P 5/48

(54) **STABLE PEPTIDE FORMULATIONS AND METHODS FOR PREPARATION**
STABILE PEPTIDFORMULIERUNGEN UND VERFAHREN ZUR HERSTELLUNG
FORMULATIONS STABLES DE PEPTIDES ET LEURS PROCÉDÉS DE PRÉPARATION

(30) Priority: 06.02.2014 US 201461936675 P
(43) Date of publication of application: 14.12.2016
(73) Proprietor: Xeris Pharmaceuticals, Inc., Austin TX 78705 (US)
(72) Inventor: PRESTRELSKI, Steven, Austin, TX 78705 (US); DONOVAN, Martin, Austin, TX 78705 (US)
(74) Representative: Uexküll & Stolberg
(86) International application number: PCT/US2015/014756
(87) International publication number: WO 2015/120231

(56) References cited:
- EP-A1- 2 526 996
- WO-A1-2013/067022
- US-A1- 2012 232 001

## Description

### BACKGROUND OF THE INVENTION

### I. Field of the Invention

The present invention relates to pharmaceutical formulations and, more particularly, to pharmaceutical formulations of peptides having improved stability and to such pharmaceutical formulations for use in methods to treat various diseases, conditions and disorders.

### II. Description of the Related Art

Diabetes is a serious health problem in modern society. Insulin is a critical treatment for both type I and type II diabetes. Studies over the past two decades have demonstrated that tight metabolic control of glucose through the use of insulin not only reduces the incidence, but also delays the development of complications in people with type 1 and type 2 diabetes. Unfortunately, the intensive insulin therapy required to achieve tight glucose control is also associated with a significantly increased risk of developing hypoglycemia or "low blood sugar."

Symptoms of hypoglycemia vary greatly among patients, but typically include tremor, palpitations, irritability, anxiety, nervousness, hunger, tachycardia, headache and pallor. The symptoms typically subside once plasma glucose is restored to normal levels. If hypoglycemia is not reversed, a further decrease in plasma glucose can lead to depletion of glucose in the central nervous system and associated neuroglycopenic symptoms, such as difficulty in concentration, slurred speech, blurred vision, reduction in body temperature, behavioral changes and, if not treated, unconsciousness, seizure and possibly death.

In general, hypoglycemia can be defined as minor to moderate hypoglycemia or as severe hypoglycemia as follows:
Minor to moderate hypoglycemia: Episodes that the patient can self-treat, regardless of the severity of symptoms, or any asymptomatic blood glucose measurements in which blood glucose levels are less than 70 mg/dL (3.9 mmol/L).
Severe hypoglycemia: Operationally defined as an episode of hypoglycemia that the patient cannot self-treat so that external help is required. Typically, neuroglycopenic symptoms and cognitive impairment begin at a blood glucose level of about 50 mg/dL (2.8 mmol/L).

Most episodes of minor to moderate hypoglycemia can be self-treated relatively easily by ingesting fast-acting carbohydrates such as glucose tablets or food (juice, soft drinks or sugary snacks). Severe hypoglycemia, by definition, cannot be self-treated and thus requires external intervention. If the patient can swallow and is cooperative, it is appropriate to use gels or products such as honey or jelly placed inside the cheek. If the patient is unable to swallow, glucagon, which is injected subcutaneously or intramuscularly, is used to treat severe hypoglycemia.

Glucagon is a naturally occurring peptide hormone that is 29 amino acids in length and is secreted by the α-cells of the pancreas. The principal function of glucagon is to maintain glucose production through both glycogenolysis and gluconeogenesis, mostly mediated via the liver. Glucagon is the primary counter-regulatory hormone to insulin and is used as a first-line treatment of severe hypoglycemia in patients with diabetes.

Numerous attempts have been made to create a glucagon rescue medication for treating severe hypoglycemia in emergency situations. Currently, there are two glucagon kits currently available in the United States, manufactured by Eli Lilly (Glucagon Emergency Kit) and Novo Nordisk (GlucaGen® HypoKit). Both products combine a vial of freeze-dried glucagon with a pre-filled syringe of aqueous diluent. The freeze-dried glucagon must be reconstituted using a complex procedure that is difficult to use in an emergency situation. These products also provide a large volume injection because glucagon is poorly soluble in water. Recently, attempts have been made to improve the stability of glucagon in an aqueous solution, to create more stable glucagon analogs and/or to improve delivery of glucagon via powder injection.
US 2012/232001 A1 discloses a stable formulation for parenteral injection comprising a peptide that has been dried in a non-volatile buffer, wherein the peptide may be glucagon.
WO 2013/067022 A1 discloses the preparation of stable formulations in the presence of a non-volatile buffer, wherein the buffer is preferably a citrate-based buffer system.
EP 2 526 996 A1 discloses a process in which the human growth hormone ('hGh') is dried in the presence of Tris buffer, *i.e.,* a non-volatile buffer, yielding hGh/Tris as well as an additional a process in which citric acid is used to adjust the pH, *i.e.,* a non-volatile buffer.

Although some progress has been made, there still remains a need for a more user-friendly glucagon rescue medication for treating severe hypoglycemia in emergency situations. Such a glucagon rescue medication would need to be carried continuously by diabetics and/or their caregivers and, thus, would need to be stable at non-refrigerated temperatures (25-30 °C) for extended periods (>2 years). Ideally, it would also need to be simple to administer for the general population, and not require excessive processing/reconstitution prior to administration to the hypoglycemic patient. The glucagon rescue medication would also need to be functional over a range of temperatures, including temperatures ranging from 0 °C to 30 °C.

### SUMMARY OF THE INVENTION

To address such needs and others, the present invention provides a stable glucagon rescue formulation according to claim 1, a method of making said formulation according to claim 14 and a stable formulation of any of claims 1 to 13 or made according to claim 14 for use as a medicament, as well as this stable glucagon formulation for use in a method to treat minor, moderate, or severe hypoglycemia. Advantageously, the glucagon is stabilized in the formulations of the present invention so as to allow for long-term storage and/or delivery over a prolonged period of time. As such, the glucagon formulations of the present invention are stable at non-refrigerated temperatures (e.g. room temperature such as 25 to 30 °C) for extended periods of time, are simple to administer, without the need for reconstitution, and are functional over a range of temperatures, including temperatures ranging from 0 to 30 °C or from 0 to -30 °C, preferably from 0 to 30 °C.

Notably, the formulation technology of the present invention is a platform formulation technology and, as such, is widely applicable for the delivery of a number of therapeutic peptides that, like glucagon, have poor or limited stability and solubility in an aqueous environment. In particular, the formulation of peptides with an aprotic polar solvent such as DMSO, NMP, ethyl acetate, or a mixture thereof into high concentration, non-aqueous solutions is a valuable delivery platform for the important class of peptide therapeutics. Additionally, the formulation technology of the present invention is widely applicable for the delivery of two or more peptides in the same solution, thereby enabling the preparation of combination drug delivery formulations.

In one aspect of the present invention there is disclosed a stable formulation for parenteral injection. Alternatively, transdermal delivery such as through topical application to skin can be used. The formulation can include: (a) a peptide or a salt thereof that has been previously dried from an aqueous composition comprising a strong acid, wherein the dried peptide or salt thereof has a first ionization profile that corresponds to the peptide's optimal stability and solubility; and an aprotic polar solvent, wherein the dried peptide or salt thereof is reconstituted into an aprotic polar solvent and has a second ionization profile in the aprotic polar solvent, wherein the first and second ionization profiles are substantially the same, such as within 1 pH unit of one another. One non-limiting method for measuring the ionization state of the dry peptide includes reconstituting the dried peptide into un-buffered water and measuring the pH of the reconstituted peptide with a pH indicator such as pH paper or a calibrated pH electrode. One non-limiting method for measuring the ionization state of the peptide that has been reconstituted in the aprotic polar solvent includes adding at least 20% water to the aprotic polar solvent and measuring the pH with a pH indicator. The peptide or salt thereof can have a third ionization profile when the peptide is in the aqueous composition prior to the aforementioned drying step. The third ionization profile can be different from the first or second ionization profiles by at least 1 pH unit (e.g., the aqueous composition can be formulated such that the pH of the aqueous composition compensates for the loss of counter-ions or buffer components or both during drying of said aqueous composition). Alternatively, the third ionization profile can be substantially the same as the first or second ionization profiles, such as within 1 pH unit of one another. One non-limiting method for measuring the ionization state of the peptide in the aqueous composition prior to said drying step is to measure the pH of the aqueous solution with a pH indicator. In some particular aspects, the aqueous composition is formulated such that the third ionization profile shifts to the first ionization profile during drying of said aqueous composition. The dried peptide can be partially or fully solubilized within the aprotic polar solvent. Full solubilization can be obtained by adding the dried peptide to the aprotic polar solvent up to the solubility limit of said peptide. For partial solubilization, suspensions and pastes can be formed such that a percentage of the peptide is solubilized in the aprotic polar solvent and a percentage is suspended or dispersed within said aprotic polar solvent. The aqueous composition can include a partially volatile buffer, non-limiting examples of which include sodium acetate or ammonium phosphate or any combination thereof. The aqueous composition can include a volatile buffer, non-limiting examples of which include ammonium acetate, ammonium formate, ammonium carbonate, ammonium bicarbonate, pyridine acetate, pyridine formate, or triethylammonium acetate, or any combination thereof. The aqueous composition includes a strong acid, a non-limiting example of which includes hydrochloric acid. The aqueous composition can include a strong base, non-limiting examples of which include sodium hydroxide, potassium hydroxide, lithium hydroxide, or calcium hydroxide, or any combination thereof. In certain aspects, the aqueous composition does not include any buffer. According to present invention, the aqueous composition does not include a non-volatile buffer. The aqueous composition can include a mixture of different buffers. In one non-liming aspect, the mixture can include a a mixture of partially volatile buffers, a mixture of volatile buffers, or a mixture of partially volatile and volatile buffers. The drying step can be performed by lyophilization, spray drying, desiccation, thin-film freezing, spray freeze drying, or any combination thereof. The moisture or water content of the formulation can be less than 15%, 10%, 5%, 1%, or less. Non-limiting examples of aprotic polar solvents includes dimethylsulfoxide (DMSO), n-methyl pyrrolidone (NMP), ethyl acetate, dimethylformamide (DMF), propylene carbonate, or mixtures thereof. The formulation can further include a co-solvent that depresses the freezing point of the formulation (e.g., ethanol, propylene glycol, glycerol, and mixtures thereof). The formulation can further include a stabilizing excipient (e.g., a sugar, a starch, or mixtures thereof). According to present invention, the peptide in the formulation is glucagon or a salt thereof. In instances where the peptide is glucagon or a salt thereof, the first or second ionization profiles can correspond to the ionization profile of glucagon when solubilized in an aqueous solution having a pH range of about 2 to 3. The third ionization profile can correspond to the ionization profile of glucagon when solubilized in an aqueous solution having a pH range of about 2 to 3 or can correspond to the ionization profile of glucagon when solubilized in an aqueous solution having a pH range of greater than 3, or greater than 3 to 14, or greater than 3 to 10, or greater than 3, 4, 5, 6, 7, 8, 9, 10, 11, 12, 13, or 14 or any range therein. Alternatively, the third ionization profile can correspond to the ionization profile of glucagon when solubilized in an aqueous solution having a pH range of less than 2, or less than 2 to 0 or less than 2 to 1 or 1 or 0 or any range therein. It is also disclosed that the first ionization profile can be maintained by reconstituting the dried peptide or salt thereof in an organic solvent system comprising an organic solvent and an organic phase buffer prior to reconstituting said dried peptide or salt thereof into polar aprotic solvent. The dried peptide or salt thereof can be reconstituted into the polar aprotic solvent with mixing the organic solvent system with the polar aprotic solvent. The organic solvent system can be separated from the polar aprotic solvent via separation methods known in the art. The organic solvent system can be substantially anhydrous (e.g., less than 1 wt. %, less than 0.5 wt. %, or less than 0.1 wt. % water) or anhydrous. The stable formulation can be comprised in a syringe, a pen injection device, an auto-injector device, or a pump device. The inventive concept can also be used with peptides other than glucagon, non-limiting examples of which include pramlintide, insulin, leuprolide, an LHRH agonist, parathyroid hormone (PTH), amylin, botulinum toxin, hematide, an amyloid peptide, cholecystikinin, a conotoxin, a gastric inhibitory peptide, an insulin-like growth factor, a growth hormone releasing factor, an anti-microbial factor, glatiramer, glucagon-like peptide-1 (GLP-1), a GLP-1 agonist, exenatide, analogs thereof, and mixtures thereof. Further, the formulation can include at least 2, 3, 4, or more different peptides, with each peptide having an ionization profile that corresponds to its optimum stability and solubility. In some embodiments, the stable formulation can be co-formulated with at least a second, third, fourth, and/or fifth peptide or salts thereof. The second, third, fourth, or fifth peptides or salts thereof can each be different from one another and from the first peptide. The second, third, fourth, or fifth peptides or salts thereof can be dried from second, third, fourth, or fifth aqueous solutions or compositions, wherein the second, third, fourth, or fifth dried peptide each have an ionization profile that is about equal to the ionization profile of the second, third, fourth, or fifth peptide when said second, third, fourth, or fifth peptide is dissolved in the second, third, fourth, or fifth aqueous solution or composition. The pH of said second, third, fourth, or fifth aqueous solution or composition can be about equal to a pH of optimal stability and solubility for the second, third, fourth, or fifth peptide. The second, third, fourth, or fifth peptide can be partially or fully solubilized in the aprotic polar solvent.

In still another aspect, the formulations of the present invention can be made by: (a) drying an aqueous composition comprising a peptide or a salt thereof and a strong acid to a dried peptide powder, wherein the dried peptide powder has a first ionization profile that corresponds to the peptide's optimal stability and solubility; and (b) reconstituting the dried peptide powder in an aprotic polar solvent, wherein the reconstituted dried peptide powder has second ionization profile in the aprotic polar solvent, wherein the first and second ionization profiles are within 1 pH unit of one another. The peptide or salt thereof can have a third ionization profile in the aqueous composition prior to drying, wherein the third ionization profile is different from the first or second ionization profiles by at least 1 pH unit. The aqueous composition is formulated such that the third ionization profile shifts to the first ionization profile upon drying of said aqueous composition. The peptide or salt thereof can have a third ionization profile in the aqueous composition prior to drying, wherein the third ionization profile is substantially the same as the first or second ionization profiles, such as within 1 pH unit of one another. The aqueous composition can be formulated such that the pH of the aqueous composition compensates for the loss of counter-ions or buffer components or both upon drying of said aqueous composition. The peptide or salt thereof is glucagon, and the aqueous composition can have a pH range of 2 to 3 prior to drying and wherein the first or second or both ionization profiles correspond to the ionization profile of glucagon when solubilized in said aqueous composition. The third ionization profile can correspond to the ionization profile of glucagon when solubilized in the aqueous composition. The aqueous composition has a pH range of greater than 3 to 7 prior to drying and wherein the first or second or both ionization profiles correspond to the ionization profile of glucagon when solubilized in an aqueous solution having a pH range of 2 to 3. It is also disclosed that prior to step (b), the dried peptide powder can be reconstituted in an organic solvent system comprising an organic solvent and an organic phase buffer, wherein the pH of the organic phase buffer is set such that the peptide powder maintains its first ionization profile or such that the peptide powder's ionization profile is re-adjusted to its first ionization profile. The organic solvent system can be combined or mixed with the aprotic polar solvent such that a two-phased system is obtained, wherein the first phase comprises the polar aprotic solvent and at least a portion of the suspended or partially suspended dried peptide powder and the second phase comprises the organic solvent system having the organic solvent and the organic buffer. The organic solvent system can be substantially anhydrous or anhydrous. Non-limiting examples of organic solvents include hexane, heptane, dodecane, hexadecane, ethyl ether, isopropyl ether, butyl ether, acetonitrile, tetrahydrofuran, dioxane, toluene, pyridine, acetone, 2-pentanone, 2-heptanine, or any combination thereof. Non-limiting examples of organic phase buffer include triphenylacetic acid, triisooctylamine, or any combination thereof.

Also disclosed is a stable formulation for parenteral injection or transdermal administration comprising: (a) a peptide or a salt thereof that has a first ionization profile corresponding to the peptide's optimal stability and solubility, wherein said first ionization profile has been set with an organic solvent system comprising an organic solvent and an organic phase buffer; and (b) an aprotic polar solvent comprising the peptide or salt thereof, wherein the peptide or salt thereof has a second ionization profile in the aprotic polar solvent, wherein the first and second ionization profiles are substantially the same, such as within 1 pH unit of one another. The peptide or salt thereof from (a) can be previously dried from an aqueous composition comprising a partially volatile buffer, a volatile buffer, a strong acid, a strong base, or a non-volatile buffer, or any combination thereof. The stable formulation can include at least two phases, the first phase comprising the aprotic polar solvent comprising the peptide or salt thereof, and the second phase comprising the organic solvent system. The organic solvent system can be substantially anhydrous or anhydrous. Non-limiting examples of organic solvents include hexane, heptane, dodecane, hexadecane, ethyl ether, isopropyl ether, butyl ether, acetonitrile, tetrahydrofuran, dioxane, toluene, pyridine, acetone, 2-pentanone, 2-heptanine, or any combination thereof. Non-limiting examples of organic phase buffer include triphenylacetic acid, triisooctylamine, or any combination thereof. The stable formulation can be comprised in a syringe, a pen injection device, an auto-injector device, or a pump device. Further, the formulation can include a second peptide or a salt thereof (or a third or fourth or fifth or more peptides). The additional peptides can de different from the first peptide. The second (as well as third, fourth, fifth, or more peptides) can have a third ionization profile corresponding to the second peptide's optimal stability and solubility, wherein said third ionization profile has been set with a second organic solvent system comprising a second organic solvent and a second organic phase buffer.

In another aspect, there is disclosed a kit comprising: (a) a first composition comprising an organic solvent system that includes an organic solvent, an organic phase buffer, and a peptide or salt thereof having a first ionization profile that corresponds to the peptide's optimal stability and solubility; and (b) a second composition comprising an aprotic polar solvent capable of partially or fully solubilizing the peptide or salt thereof and maintaining the first ionization profile. The first and second compositions can be included in first and second containers, respectively. The peptide or salt thereof can be such that it has been previously dried from an aqueous composition comprising a partially volatile buffer, a volatile buffer, a strong acid, a strong base, or a non-volatile buffer, or any combination thereof. The first ionization profile can be obtained from drying said peptide or salt thereof from said aqueous solution. The first ionization profile can be obtained from the first composition. The peptide or salt thereof can be suspended or partially suspended in the organic solvent system. The first composition can be substantially anhydrous or anhydrous.

In another aspect, there is disclosed a stable formulation for parenteral injection, the formulation comprising: (a) a peptide or a salt thereof, wherein the peptide has been dried to a powder from an aqueous solution where the pH has been adjusted so as to produce a peptide-containing powder upon drying (e.g. via lyophilization) wherein the ionization state of the peptide in the powder is about equal to the ionization state of the peptide in an aqueous solution wherein the pH of said aqueous solution is about equal a pH of optimal stability and solubility for the peptide, and (b) an aprotic polar solvent, wherein the peptide, once reconstituted in the aprotic polar solvent maintains the ionization state that is about equal to the ionization state of the peptide in the powder.

In still another aspect, there is disclosed a stable formulation for parenteral injection, the formulation comprising: (a) a first peptide or a salt thereof, wherein the first peptide has been dried from an aqueous solution, and wherein the first dried peptide has an ionization profile that is about equal to the ionization profile of the peptide when it is dissolved in an aqueous solution wherein the pH of said aqueous solution is about equal to a pH of optimal stability and solubility for the first peptide; (b) a second peptide or a salt thereof, wherein the second peptide has been dried from a second aqueous solution, and wherein the second dried peptide has an ionization profile that is about equal to the ionization profile of the peptide when it is dissolved in an aqueous solution wherein the pH of said aqueous solution is about equal to a pH of optimal stability and solubility for the second peptide; and (c) an aprotic polar solvent. The moisture or water content of the formulation can be less than 15%, 10%, 5%, 1%, or less. The first peptide, following reconstitution in the aprotic polar solvent, maintains the first ionization profile that is about equal to the ionization profile of first peptide powder, and wherein the second dried peptide, following reconstitution in the aprotic polar solvent, maintains the second ionization profile that is about equal to the ionization profile of the second peptide powder. Third, fourth, fifth, or more peptides can also be co-formulated with the first and second peptides. Each peptide can be different (e.g., the first can be different from the second, third, fourth, and fifth peptides, the second can be different from the third, fourth, and fifth peptides, etc.). Also, the additional peptides co-formulated with the first peptide can be partially or fully solubilized in the aprotic polar solvent.

In yet another aspect, there is disclosed a stable formulation for parenteral injection, the formulation comprising: a peptide or a salt thereof (such as a hydrochloride or acetate salt thereof); and an aprotic polar solvent, wherein the moisture content of the formulation is less than 15, 10, 5, 1% or less.

The stable formulations described herein are useful for the parenteral injection of any peptide that has limited or poor stability or solubility in an aqueous environment. Thus, in some disclosures the peptide (or each of the first and second peptides) or salt thereof is selected from the group consisting of glucagon, pramlintide, insulin, leuprolide, an LHRH agonist, parathyroid hormone (PTH), amylin, botulinum toxin, hematide, an amyloid peptide, cholecystikinin, a conotoxin, a gastric inhibitory peptide, an insulin-like growth factor, a growth hormone releasing factor, an anti-microbial factor, glatiramer, glucagon-like peptide-1 (GLP-1), a GLP-1 agonist, exenatide, analogs thereof, and mixtures thereof. The peptide of the invention is glucagon. In an embodiment, the first peptide is glucagon and the second peptide is exenatide.

In certain aspects, the peptide (or, in embodiments where the formulation comprises two or more peptides, each of the peptides) can be dissolved in an aqueous solution, and dried to a dry peptide powder, and then reconstituted in an aprotic polar solvent. The pH of the aqueous solution from which the peptide is dried can be adjusted such that upon drying (e.g. via lyophilization) the peptide in the powder has an ionization state that is about equal to the ionization state of the peptide in an aqueous solution wherein the pH of said aqueous solution is about equal to a pH of optimal stability and solubility for the peptide. The aqueous solution from which the peptide is dried may include one or more buffers, wherein the buffers may be partially volatile buffers, volatile buffers, or mixtures thereof.

When the aqueous solution includes a non-volatile buffer not according to the present invention, the ionization state of the peptide in the aqueous solution may be about equal to the ionization state of the peptide in the powder (i.e., "about equal" refers to when the ionization profile of the peptide dried from an aqueous solution of pH X corresponds to the ionization profile of the peptide in an aqueous solution wherein the difference between the pH of said aqueous solution and pH X is within (or equal to) 1 pH unit). Suitable non-volatile buffers include, but are not limited to, glycine buffers, citrate buffers, phosphate buffers, and mixtures thereof. In one preferred embodiment, the non-volatile buffer is a glycine buffer. In another preferred embodiment, the non-volatile buffer is a mixture of citrate buffer and phosphate buffer. In some embodiments, wherein the formulation comprises two or more peptides, the first non-volatile buffer and the second non-volatile buffer are the same. In some embodiments, wherein the formulation comprises two or more peptides, the first non-volatile buffer and the second non-volatile buffer are different. When the aqueous solution includes a partially volatile, or volatile buffer, the ionization state of the peptide in the aqueous solution may not be equal to about the ionization state of the peptide in the powder (i.e., "not equal" refers to when the ionization profile of the peptide dried from an aqueous solution of pH X corresponds to the ionization profile of the peptide in an aqueous solution where the difference between the pH of said aqueous solution and pH X is greater than 1 pH unit. In some embodiments, wherein the formulation comprises two or more peptides, the first buffer and the second buffer are the same. In some embodiments, wherein the formulation comprises two or more peptides, the first buffer and the second buffer are different. When the aqueous solution from which a peptide is dried does not include a buffer, and the pH is adjusted using a strong acid and/or strong base (e.g. HCl and/or NaOH) the ionization state of the peptide in the powder may, or may not, be about equal to the ionization state of the peptide in the aqueous solution from which the peptide was dried. Accordingly, dependent upon the components of the aqueous solution from which a peptide is dried, the ionization state of the peptide in the powder may, or may not, be about equal to the ionization state of the peptide in the aqueous solution from which it was dried.

It is also disclosed that the ionization state of the peptide may be adjusted following reconstitution of the dried peptide in an organic solvent (e.g. hexane, heptane) through the use of organic phase buffers (prepared from compounds that are sufficiently hydrophobic that even the ionized form is soluble in the organic phase). Non-limiting examples of such compounds include triphenylacetic acid and triisooctylamine, as described in Blackwood, et al, 1994 (Blackwood, et al. (1994) 'Organic phase buffers' control biocatalyst activity independent of initial aqueous pH, Biochimica et Biophysica Acta, Volume 1206, pages 161 - 165). The organic solvent can be replaced with an aprotic polar solvent such as DMSO, wherein the peptide may partition into the aprotic polar solvent and the organic phase buffer may partition into the more hydrophobic organic solvent. In such an embodiment, the ionization state of the peptide in the powder may not be about equal to the ionization state of the peptide in an aqueous solution where the pH of said aqueous solution is about equal to a pH of optimal stability and solubility for the peptide. Following reconstitution in the organic solvent (e.g. hexane, heptane) the ionization state of the peptide may be adjusted using organic phase buffers to be about equal to the ionization state of the peptide in an aqueous solution wherein the pH of said aqueous solution is about equal to a pH of optimal stability and solubility for the peptide through the use of organic phase buffers.

In some formulations of the present invention, the peptide is mixed with a stabilizing excipient, and then dried to a dry peptide powder. Suitable stabilizing excipients include, but are not limited to, sugars, starches, and mixtures thereof. In some embodiments, the sugar is trehalose. In some embodiments, the starch is hydroxyethyl starch (HES). In some embodiments, the stabilizing excipient is present in the formulation in an amount ranging from about 1% (w/v) to about 60% (w/v), from about 1% (w/v) to about 50% (w/v), from about 1% (w/v) to about 40% (w/v), from about 1% (w/v) to about 30% (w/v), from about 1% (w/v) to about 20% (w/v), from about 5% (w/v) to about 60% (w/v), from about 5% (w/v) to about 50% (w/v), from about 5% (w/v) to about 40% (w/v), from about 5% (w/v) to about 30% (w/v), from about 5% (w/v) to about 20% (w/v), from about 10% (w/v) to about 60% (w/v), from about 10% (w/v) to about 50% (w/v), from about 10% (w/v) to about 40% (w/v), from about 10% (w/v) to about 30% (w/v), or from about 10% (w/v) to about 20% (w/v). In some embodiments, wherein the formulation comprises two peptides, both of the first peptide in the first aqueous solution and the second peptide in the second aqueous solution further comprise a stabilizing excipient, and the stabilizing excipient with the first peptide in the first aqueous solution and the stabilizing excipient with the second peptide in the second aqueous solution are the same. In other embodiments, wherein the formulation comprises two peptides, both the first peptide in the first aqueous solution and the second peptide in the second aqueous solution further comprise a stabilizing excipient, and the stabilizing excipient with the first peptide in the first aqueous solution and the stabilizing excipient with the second peptide in the second aqueous solution are different.

Once the peptide or peptides, potentially including excipients as discussed above are dried to a powder, the dried peptide powder is dissolved or reconstituted in an aprotic polar solvent. Examples of aprotic polar solvents include, but are not limited to, the following: dimethylsulfoxide (DMSO), dimethylformamide (DMF), ethyl acetate, n-methyl pyrrolidone (NMP), dimethylacetamide (DMA), propylene carbonate, and mixtures thereof. Dimethylsulfoxide (DMSO), n-methyl pyrrolidone (NMP), ethyl acetate, and mixtures of one or more of DMSO, NMP, and ethyl acetate are particularly preferred aprotic polar solvents. In a preferred embodiment, the aprotic polar solvent is DMSO. In another preferred embodiment, the aprotic polar solvent is a mixture of DMSO and NMP. In yet another preferred embodiment, the aprotic polar solvent is a mixture of DMSO and ethyl acetate.

In some embodiments, the peptide or peptides are reconstituted in a mixture of an aprotic polar solvent (e.g., dimethylsulfoxide (DMSO), dimethylformamide (DMF), ethyl acetate, n-methyl pyrrolidone (NMP), dimethylacetamide (DMA), propylene carbonate, or mixtures thereof) and a co-solvent that depresses the freezing point of the formulation. In some embodiments, the co-solvent depresses the freezing point of the formulation by at least about 5 °C., at least about 10 °C, at least about 15 °C, or at least about 20 °C. In some embodiments, the co-solvent depresses the freezing point of the formulation to about 3 °C to about 2 °C to about 1 °C or to about 0 °C or below. In some embodiments, the co-solvent is a polar protic solvent. In preferred embodiments, the co-solvent is selected from ethanol, propylene glycol (PG), glycerol, and mixtures thereof. In some embodiments, the co-solvent is present in the formulation in an amount ranging from about 10% (w/v) to about 50% (w/v), from about 10% (w/v) to about 40% (w/v), from about 10% (w/v) to about 30% (w/v), from about 10% (w/v) to about 25% (w/v), from about 15% (w/v) to about 50% (w/v), from about 15% (w/v) to about 40% (w/v), from about 15% (w/v) to about 30% (w/v), or from about 15% (w/v) to about 25% (w/v).

The pH of the aqueous solution from which the peptide is dried can be selected so as to yield a powder wherein the peptide in the powder has an ionization profile that is about equal to the ionization profile of the peptide in an aqueous solution wherein the pH of said aqueous solution is about equal to a pH of optimal stability and solubility for the peptide. When this peptide powder is reconstituted in an aprotic polar solvent (e.g. DMSO), the ionization profile of the peptide is about equal to the ionization state of the peptide in the powder. As such, in preferred embodiments, the ionization state of the peptide in the formulation should be about equal to the ionization state of the peptide in an aqueous solution wherein the pH is about 2.0 to about 3.0 to ensure optimal stability, maximal solubility, and minimal degradation. In other embodiments, the ionization state of the peptide in the formulation should be about equal to the ionization state of the peptide in an aqueous solution wherein the pH of said aqueous solution is about 3.0 to about 5.0 to ensure optimal stability, maximal solubility, and minimal degradation. In other embodiments, the ionization state of the peptide in the formulation should be about equal to the ionization state of the peptide in an aqueous solution wherein the pH of said aqueous solution is about 4.0 to about 5.0 to ensure optimal stability, maximal solubility, and minimal degradation. In yet other embodiments, the ionization state of the peptide in the formulation should be about equal to the ionization state of the peptide in an aqueous solution wherein the pH of said aqueous solution is about 4.0 to about 6.0 to ensure optimal stability, maximal solubility, and minimal degradation. In yet other embodiments, the ionization state of the peptide in the formulation should be about equal to the ionization state of the peptide in an aqueous solution wherein the pH of said aqueous solution is about 6.0 to about 8.0 to ensure optimal stability, maximal solubility, and minimal degradation. In some embodiments, wherein the formulation comprises two peptides, the ionization state of the first peptide in the formulation should be about equal to the ionization state of the peptide in an aqueous solution wherein the pH of said aqueous solution is about 4.0 to about 6.0 to ensure optimal stability, maximal solubility, and minimal degradation, and the ionization state of the second peptide should be about equal to the ionization state of the peptide in an aqueous solution wherein the pH of said aqueous solution is about 1.5 to about 2.5, or of about 6.0 to about 8.0, to ensure optimal stability, maximal solubility, and minimal degradation. In some embodiments, wherein the formulation comprises two peptides, the ionization state of the first peptide in the formulation should be about equal to the ionization state of the peptide in an aqueous solution wherein the pH of said aqueous solution is about 3.0 to about 5.0 to ensure optimal stability, maximal solubility, and minimal degradation, and the ionization state of the second peptide should be about equal to the ionization state of the peptide in an aqueous solution wherein the pH of said aqueous solution is about 1.5 to about 2.5, or of about 6.0 to about 8.0, to ensure optimal stability, maximal solubility, and minimal degradation. In other embodiments, wherein the formulation comprises two peptides, the ionization state of the first peptide should be about equal to the ionization state of the peptide in an aqueous solution wherein the pH of said aqueous solution is about 2.0 to about 3.0 to ensure optimal stability, maximal solubility, and minimal degradation, and the ionization state of the second peptide should be about equal to the ionization state of the peptide in an aqueous solution wherein the pH of said aqueous solution is about 4.0 to about 5.0 to ensure optimal stability, maximal solubility, and minimal degradation. It will be readily apparent to one of skill in the art how to determine the optimal pH for obtaining a peptide having optimal stability, maximal solubility, and minimal degradation.

Any suitable dosage of peptide or peptides can be formulated in the stable formulations of the present invention. Generally, the peptide (or, in embodiments comprising two or more peptides, each of the peptides) is present in the formulation in an amount ranging from about 0.5 mg/mL to about 100 mg/mL. In some embodiments, the peptide is present in the formulation in an amount ranging from about 10 mg/mL to about 60 mg/mL. In other embodiments, the peptide is present in the formulation in an amount ranging from about 20 mg/mL to about 50 mg/mL. In still other embodiments, the peptide is present in the formulation in an amount ranging from about 5 mg/mL to about 15 mg/mL. In yet other embodiments, the peptide is present in the formulation in an amount ranging from about 0.5 mg/mL to about 2 mg/mL. In yet other embodiments, the peptide is present in the formulation in an amount ranging from about 1 mg/mL to about 50 mg/mL. Again, it will be readily apparent to those of skill that the peptide dosage can be varied depending on the peptide used and the disease, disorder or condition to be treated.

In some embodiments, the formulations of the present invention further comprise an antioxidant. In other embodiments, the formulations further comprise a chelator. In still other embodiments, the formulations of the present invention further comprise a preservative.

In another aspect, the present invention provides a formulation for use in a method for treating a disease, condition or disorder that may be treated, alleviated, or prevented by administering to a subject a stable peptide formulation as described herein in an amount effective to treat, alleviate or prevent the disease, condition, or disorder. In some embodiments, the disease, condition, or disorder is hypoglycemia. In some embodiments, wherein the disease, condition, or disorder is hypoglycemia, the method comprises administering a stable glucagon formulation of the present invention in an amount effective to treat the hypoglycemia. In some embodiments, the disease, condition, or disorder is diabetes. In other aspects, the disease, condition, or disorder is congenital hyperinsulinism, insulinoma, or any other disorder where excess levels of insulin can induce hypoglycemia.

There is disclosed a process for making a stable formulation for parenteral injection, the process comprising: drying a peptide from an aqueous solution to a dry peptide powder wherein the peptide in the powder has an ionization profile that is about equal to the ionization profile of the peptide in an aqueous solution wherein the pH of said aqueous solution is about equal to a pH of optimal stability and solubility for the peptide; and reconstituting the dry peptide powder with an aprotic polar solvent, thereby making the stable formulation, wherein the moisture content of the stable formulation is less than 5%. In some disclosures the dried peptide powder has an ionization profile that is about equal to the ionization profile of the peptide in the aqueous solution from which it was dried, and the dried peptide maintains the ionization profile that is about equal to the ionization profile of the peptide in the aqueous solution from which it was dried when the dried peptide powder is reconstituted in the aprotic polar solvent. In other disclosures, the dried peptide powder has an ionization profile that is not about equal to the ionization profile of the peptide in the aqueous solution from which it was dried, and the dried peptide powder maintains the ionization profile when the dried peptide powder is reconstituted in the aprotic polar solvent.

Still another disclosure provides kits for treating a disease, condition or disorder, the kit comprising: a stable formulation comprising one or more peptides or salts thereof, wherein the peptide(s) has been dried to a powder wherein the peptide in the powder has an ionization profile that is about equal to the ionization profile of the peptide in an aqueous solution wherein the pH of said aqueous solution is about equal to a pH of optimal stability and solubility for the peptide; and an aprotic polar solvent; wherein the moisture content of the formulation is less than 5%, and wherein the dried peptide(s) maintains the ionization profile that is about equal to the ionization profile of the peptide in an aqueous solution wherein the pH of said aqueous solution is about equal to a pH of optimal stability and solubility for the peptide when the dried peptide(s) is reconstituted in the aprotic polar solvent; and a syringe for administration of the stable formulation to the subject.

In some disclosures, the kit is for treating hypoglycemia and the stable formulation comprises a glucagon formulation as described herein. In some disclosures the kit is for treating diabetes and the stable formulation comprises an insulin and pramlintide formulation as described herein. In some disclosures, the syringe is part of a pen injection device, an auto-injector device or a pump. In some disclosures, the syringe is prefilled with the stable formulation. In some disclosures, the kit further comprises instructions, wherein the instructions direct the administration of the stable formulation to treat the subject in need thereof.

Prior to administration of the composition, the patient can have mild or moderate hypoglycemia. In some instances, the patient can have severe hypoglycemia (e.g., unable to self-treat or administer such that external help is required (e.g., health care provider such as a doctor or nurse or a mechanical device such as a pump (e.g., bi-hormonal pump)) or be diagnosed as having a blood glucose level of less than 70, 60, 50, 40, 30, 20, 10, 5, or 0 mg/dL or be diagnosed as having a blood glucose level between 0 to 10 mg/dL, 10 to 20 mg/dL, 20 to 30 mg/dL, 30 to 40 mg/dL, 40 to 50 mg/dL, 50 to 60 mg/dL, or 60 to 70 mg/dL, or be diagnosed as having a blood glucose level between 5 to 70 mg/dL, 5 to 60 mg/dL, 5 to 50 mg/dL, 5 to 40 mg/dL, 5 to 30 mg/dL, 5 to 20 mg/dL, or 5 to 10 mg/dL, or be diagnosed as having a blood glucose level of between 10 to 70 mg/dL, 10 to 60 mg/dL, 10 to 50 mg/dL, 10 to 40 mg/dL, or 10 to 30 mg/dL, or be diagnosed as having a blood glucose level of 20 to 70 mg/dL, 20 to 60 mg/dL, 20 to 50 mg/dL, or 20 to 40 mg/dL, or be diagnosed as having a blood glucose level of 30 to 70 mg/dL, 30 to 60 mg/dL, or 30 to 50 mg/dL. Alternatively, and in certain instances, the patient can have a blood glucose level at, around, or greater than 50, 60, 70, 80, 90, 100, 110, 120, 130, 140, 150, 160, 170, 180, 190, or 200 (or any range or integer therein) and also have an indication of impending hypoglycemia or an indication that the blood glucose levels will fall to below 70, 60, or 50 mg/dL within a certain period of time (e.g., within 30, 25, 20, 15, 10, 9, 8, 7, 6, 5, 4, 3, 2, or 1 minute(s). Such an indication can be determined by identifying a downward trend of blood glucose levels (*e.g.,* by a blood glucose monitoring device) as well as the speed or trajectory of this downward trend. In either instance (e.g., the patient is diagnosed with hypoglycemia or is at risk of developing hypoglycemia), administration of the composition to the patient can result in the patient maintaining or increasing the blood glucose level to greater than 50 mg/dL to 180 mg/dL within 1 to 20, 1 to 15, 1 to 10, 1 to 5 or within 1, 2, 3, 4, 5, 6, 7, 8, 9, 10, 11, 12, 13, 14, 15, 16, 17, 18, 19, or 20 minutes after administration of the composition. In a particular aspect, the patient can be diagnosed as having a blood glucose level between 10 mg/dL and less than 40 mg/dL prior to administering the composition. In another instance, the patient's blood glucose level can be determined to be between 50 mg/dL to 70 mg/dL based on the glucose monitoring device and trending downwards in a manner discussed above. In a particular aspect, the patient's blood glucose level can be greater than 50 mg/dL to 180 mg/dL within 1 to 10 minutes or 1 to 5 minutes after administration of the composition.

In addition to parenteral administration, the formulations of the disclosure can be transdermally delivered such as by topical application to skin. Topical application" refers to applying or spreading a composition onto the surface of keratinous tissue. "Keratinous tissue" includes keratin-containing layers disposed as the outermost protective covering of mammals and includes, but is not limited to, lips, skin, and hair. By way of example, topical application can include, but is not limited to, spreading the composition directly onto skin or loading the composition onto a substrate (e.g., bandage or dermal patch) and applying the substrate to skin.

A peptide's 'optimal stability and solubility" refers to the pH environment wherein solubility of the peptide is high (at or near the maximum on a solubility versus pH profile, or suitable for the requirements of the product) and its degradation minimized relative to other pH environments. Notably, a peptide may have more than one pH of optimal stability and solubility. A person having ordinary skill in the art can easily ascertain a given peptide's optimal stability and solubility by referencing literature or by performing assays.

"Charge profile," "charge state," "ionization state," and "ionization profile" may be used interchangeably and refer to the ionization state (i.e. due to protonation and/or deprotonation) of the peptide's ionogenic groups.

"Therapeutic agent" encompasses peptide compounds together with pharmaceutically acceptable salts thereof. Useful salts are known to those skilled in the art and include salts with inorganic acids, organic acids, inorganic bases, or organic bases. Therapeutic agents useful in the present invention are those peptide compounds that affects a desired, beneficial, and often pharmacological, effect upon administration to a human or an animal, whether alone or in combination with other pharmaceutical excipients or inert ingredients.

"Peptide," "polypeptide" and "peptide compound" refer to polymers of up to about 100 or more preferably up to about 80 amino acid residues bound together by amide (CONH) linkages. Analogs, derivatives, agonists, antagonists and pharmaceutically acceptable salts of any of the peptide compounds disclosed here are included in these terms. The terms also include peptides and peptide compounds that have D-amino acids, modified, derivatized or naturally occurring amino acids in the D- or L-configuration and/or peptomimetic units as part of their structure.

"Patient," "subject," or "individual" refers to a mammal (e.g., human, primate, dog, cat, bovine, ovine, porcine, equine, mouse, rate, hamster, rabbit, or guinea pig).

"Inhibiting" or "reducing" or any variation of these terms includes any measurable decrease or complete inhibition to achieve a desired result.

"Effective" or "treating" or "preventing" or any variation of these terms means adequate to accomplish a desired, expected, or intended result.

"Single-phase solution" refers to a solution prepared from a powder dissolved in a solvent, or solvent system (e.g., mixture of two or more solvents), wherein the particulate matter is completely dissolved in the solvent and there is no longer particulate matter visible, such that the solution can be described as optically clear.

"Buffer" refers to a weak acid or base that prevents rapid or significant changes in the pH of a solution following the addition of other acids and/or bases. When buffering agent are added to water, a buffered solution is formed. For example, a buffer solution may contain both a weak acid and its conjugate base, or a weak base and its conjugate acid. In common chemical usage, a pH buffer is a substance or a mixture of substances, which permits solutions to resist large changes in pH upon addition of small amounts of H⁺ and OH⁻ ions. A common buffer mixture contains two substances, a conjugate acid (proton donor) and a conjugate base (proton acceptor). Together, the two species (the conjugate acid-base pair of a conjugate acid and conjugate base) resist large changes in pH of the solution by partially absorbing additions of H⁺ and OH⁻ ions to the solution.

"Non-volatile buffer" refers to a buffer where the buffer components are not sufficiently volatile that they may be removed from the composition during drying (e.g., during lyophilization). Sodium phosphate is one non-limiting example of a non-volatile buffer.

"Partially-volatile buffer" refers to a buffer wherein one of the buffer components is sufficiently volatile that it may be removed from the composition during drying (e.g., during lyophilization). Ammonium phosphate is one non-limiting example of a partially-volatile buffer.

"Volatile buffer" refers to both of the buffer components being sufficiently volatile such that they may be removed from the composition during drying (e.g., during lyophilization). Ammonium formate is one non-limiting example of a volatile buffer.

"Organic phase buffer" refers to sufficiently hydrophobic weak acids/bases and their conjugate bases/acids such that even the ionized form of the buffer is soluble into the organic phase (e.g., hexane, heptane), and not be able to partition into an adjacent aqueous phase.

"Isoelectric point" (pI) of a peptide corresponds to the pH value where the overall net charge of the peptide is zero. Due to their varying composition with respect to their primary structures, peptides may have varying isoelectric points. In peptides there may be many charged groups (e.g., ionogenic groups that have been protonated or deprotonated) and at the isoelectric point the net sum of all these charges is zero, i.e. the number of negative charges balances the number of positive charges. At a pH above the isoelectric point the overall net charge of the peptide will be negative, and at pH values below the isoelectric point the overall net charge of the peptide will be positive. There are multiple methods known in the art for determining the isoelectric point of a peptide, including experimental methods such as isoelectric focusing, and theoretical methods where the isoelectric point may be estimated from the amino acid sequence of the peptide by computational algorithms.

"Reconstituted," when referring to a pharmaceutical composition, refers to a composition which has been formed by the addition of an appropriate non-aqueous solvent to a solid material comprising the active pharmaceutical ingredient. Pharmaceutical compositions for reconstitution are typically applied where a liquid composition with acceptable shelf-life cannot be produced. An example of a reconstituted pharmaceutical composition is the solution which results when adding a biocompatible aprotic polar solvent (e.g., DMSO) to a freeze dried composition.

"Primary structure" refers to the linear sequence of amino acid residues that comprise a peptide/polypeptide chain.

"Analogue" and "analog," when referring to a peptide, refers to a modified peptide wherein one or more amino acid residues of the peptide have been substituted by other amino acid residues, or wherein one or more amino acid residues have been deleted from the peptide, or wherein one or more amino acid residues have been added to the peptide, or any combination of such modifications. Such addition, deletion or substitution of amino acid residues can take place at any point, or multiple points, along the primary structure comprising the peptide, including at the N-terminal of the peptide and/or at the C-terminal of the peptide.

"Derivative," in relation to a parent peptide, refers to a chemically modified parent peptide or an analogue thereof, wherein at least one substituent is not present in the parent peptide or an analogue thereof. One such non-limiting example is a parent peptide which has been covalently modified. Typical modifications are amides, carbohydrates, alkyl groups, acyl groups, esters, pegylations and the like.

"Non-aqueous solvent" refers to a solvent that is not water.

"Aprotic polar solvent" refers to a polar solvent that does not contain acidic hydrogen and does not act as a hydrogen bond donor. Examples of aprotic polar solvents include, but are not limited to, dimethylsulfoxide (DMSO), dimethylformamide (DMF), ethyl acetate, n-methyl pyrrolidone (NMP), dimethylacetamide (DMA), and propylene carbonate. The term aprotic polar solvent also encompasses mixtures of two or more aprotic polar solvents, e.g., a mixture of two or more of dimethylsulfoxide (DMSO), dimethylformamide (DMF), ethyl acetate, n-methyl pyrrolidone (NMP), dimethylacetamide (DMA), and propylene carbonate.

"Parenteral injection" refers to the administration of small molecule drugs *via* injection under or through one or more layers of skin or mucus membranes of an animal, such as a human. Standard parenteral injections are given into the subcutaneous, intramuscular, or intradermal region of an animal, e.g., a human patient. These deep locations are targeted because the tissue expands more easily, relative to shallow dermal sites, to accommodate the 0.1-3.0 cc (mL) injection volumes required to deliver most therapeutic agents.

"Bioavailability" refers to the extent to which the therapeutic agent, such as a peptide compound, is absorbed from the formulation.

"Systemic," with respect to delivery or administration of a therapeutic agent, such as a peptide compound, to a subject, that therapeutic agent is detectable at a biologically significant level in the blood plasma of the subject.

"Controlled release" refers to the release of the therapeutic agent at such a rate that blood (e.g., plasma) concentrations are maintained within the therapeutic range, but below toxic concentrations over a period of time of about one hour or longer, preferably 12 hours or longer.

"Pharmaceutically acceptable carrier" refers to a pharmaceutically acceptable solvent, suspending agent or vehicle for delivering a drug compound of the present invention to a mammal such as an animal or human.

"Pharmaceutically acceptable" ingredient, excipient or component is one that is suitable for use with humans and/or animals without undue adverse side effects (such as toxicity, irritation and allergic response) commensurate with a reasonable benefit/risk ratio.

"Chemical stability," when referring to a therapeutic agent, such as a peptide or salt thereof, refers to an acceptable percentage of degradation products produced by chemical pathways such as oxidation or hydrolysis is formed. In particular, a formulation is considered chemically stable if no more than about 20% breakdown products are formed after one year of storage at the intended storage temperature of the product (e.g., room temperature); or storage of the product at 30° C/ 60% relative humidity for one year; or storage of the product at 40° C /75% relative humidity for one month, and preferably three months. In some embodiments, a chemically stable formulation has less than 20%, less than 15%, less than 10%, less than 5%, less than 4%, less than 3%, less than 2%, or less than 1% breakdown products formed after an extended period of storage at the intended storage temperature of the product.

"Physical stability," when referring to a therapeutic agent, such as a peptide or salt thereof, refers to an acceptable percentage of aggregates (e.g., dimers, trimers and larger forms) being formed. In particular, a formulation is considered physically stable if no more that about 15% aggregates are formed after one year of storage at the intended storage temperature of the product (e.g., room temperature); or storage of the product at 30° C / 60% relative humidity for one year; or storage of the product at 40° C / 75% relative humidity for one month, and preferably three months. In some embodiments, a physically stable formulation has less than less than 15%, less than 10%, less than 5%, less than 4%, less than 3%, less than 2%, or less than 1% aggregates formed after an extended period of storage at the intended storage temperature of the product.

"Stable formulation" refers to at least about 65% chemically and physically stable therapeutic agents, such as peptides or salts thereof, remain after two months of storage at room temperature. Particularly preferred formulations are those in which at least about 80%, 85%, 90%, 91%, 92%, 93%, 94%, 95%, 96%, 97%, 98%, or 99% chemically and physically stable therapeutic agent remains under these storage conditions. Especially preferred stable formulations are those which do not exhibit degradation after sterilizing irradiation (e.g., gamma, beta or electron beam).

The term "about" or "approximately" are defined as being close to as understood by one of ordinary skill in the art, and in one non-limiting embodiment the terms are defined to be within 10%, preferably within 5%, more preferably within 1%, and most preferably within 0.5%. Further, "substantially non-aqueous" refers to less than 5%, 4%, 3%, 2%, 1%, or less by weight or volume of water.

The use of the word "a" or "an" when used in conjunction with the term "comprising" in the claims and/or the specification may mean "one," but it is also consistent with the meaning of "one or more," "at least one," and "one or more than one."

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The compositions and methods for their use can "comprise," "consist essentially of," or "consist of' any of the ingredients or steps disclosed throughout the specification. With respect to the transitional phase "consisting essentially of," in one non-limiting aspect, a basic and novel characteristic of the formulations and methods disclosed in this specification includes the stability and solubility of the peptides or salts thereof within said formulations. Therefore, ingredients that can substantially alter the stability or solubility of the peptides or salts thereof within the formulations would be excluded from said formulations in instances where a claim uses the transitional phrase "consisting essentially of."

Other objects, features, and advantages of the present invention will be apparent to one of skill in the art from the following detailed description and claims.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

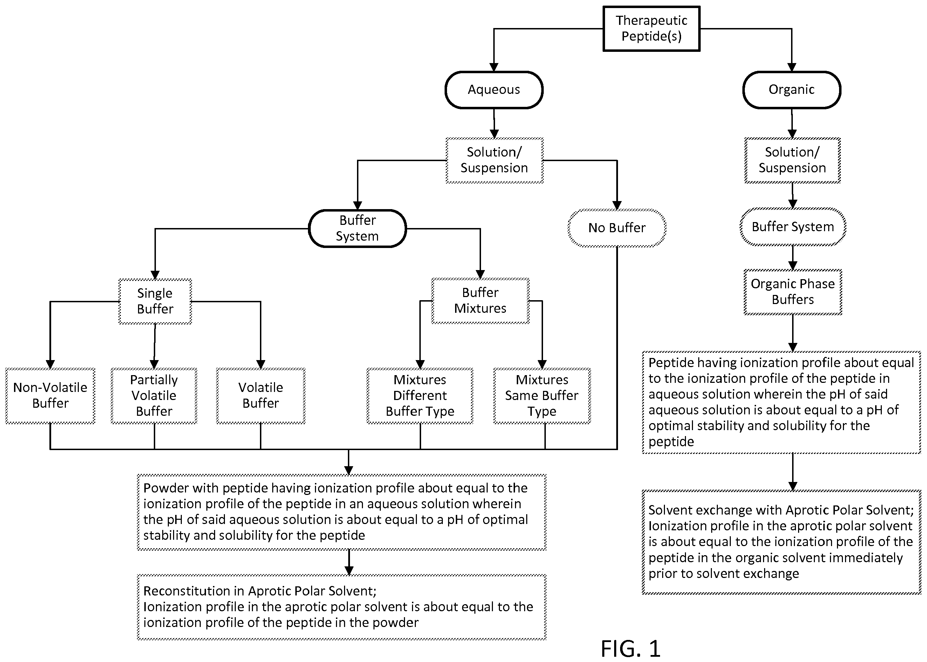

**FIG. 1** Schematic diagram of various steps that can be used to make the compositions of the present invention.

### DETAILED DESCRIPTION

Without wishing to be bound by theory, it is believed that by establishing the ionization profile of optimal stability and solubility on a peptide during drying (such that the ionogenic groups of the peptide possess this ionization state of optimal stability and solubility in the solid state (i.e. as a powder)), followed by reconstitution in an aprotic polar solvent (which will solubilize the peptide without altering the ionization state of the peptide), the ionization profile of the peptide in the aprotic polar solvent will be that of optimal stability and solubility, enabling the preparation of high concentration, thermo-stable formulations. Accordingly, the ionization profile of the peptide as a powder will be unchanged when the peptide powder is reconstituted in the aprotic polar solvent.

In aqueous solutions there may exist optimal pH where the stability and solubility of the peptide molecule in the aqueous solution is maximized. When the pH of the solution is equal to the isoelectric point (pI) of the peptide, the peptide will have no overall net charge. At a pH above the isoelectric point the overall net charge of the peptide will be negative, and at pH values below the isoelectric point the overall net charge of the peptide will be positive. The relatively long-range electrostatic repulsions between the molecules possessing overall net charge of the same sign (i.e. positive-positive, negative-negative) can prevent the shorter-range hydrophobic interactions that are often responsible for aggregation and that can lead to physical degradation via fibrillation and precipitation.

The stability and solubility of peptides in solution can increase as the pH of the aqueous solution in which they are dissolved is set away from the isoelectric point of the peptide. An example of this may be seen with the peptide glucagon, which has limited solubility at neutral pH (approximately equal to the pI of the glucagon molecule), but has increased solubility in acidic and alkaline solutions.

When the aqueous solution containing the peptide is dried to a powder (e.g. *via* lyophilization), the ionogenic groups on the peptide can be ionized. Thus, it is possible to impart an optimal ionization state to the peptide in the powder during drying such that the ionization profile of the peptide in the powder is about equal to the ionization state of the peptide in an aqueous solution wherein the pH of said aqueous solution is about equal to a pH of optimal stability and solubility for the peptide.

The ionization profile acquired by the peptide's ionogenic groups in the powder will remain when the powder is reconstituted in aprotic polar solvents, such as DMSO. Because aprotic polar solvents do not have exchangeable protons, when the dried peptide is reconstituted in an aprotic polar solvent, the reconstituted formulation may maintain the solubility and stability characteristics of an optimal ionization state of the peptide. Thus, the ionization state of the peptide in the powder influences the solubility and stability of the peptide in the aprotic polar solvent.

Accordingly, it is possible to prepare a peptide powder, wherein the ionization profile of the peptide in the powder is about equal to the ionization profile of the peptide in an aqueous solution wherein the pH of said aqueous solution is about equal to a pH of optimal stability and solubility for the peptide. When this peptide powder is reconstituted in an aprotic polar solvent, the ionization profile of the peptide in the resulting formulation will be about equal to the ionization profile of the peptide in an aqueous solution wherein the pH of said aqueous solution is about equal to a pH of optimal stability and solubility for the peptide.

### I. Introduction

Peptides can degrade via a number of different mechanisms, including deamidation, oxidation, hydrolysis, disulfide interchange and racemization. Further, water acts as a plasticizer, which facilitates unfolding of protein molecules and irreversible molecular aggregation. Therefore, in order to provide a peptide formulation that is stable over time at ambient or physiological temperatures, a non-aqueous or substantially non-aqueous peptide formulation is generally required.

Reduction of aqueous peptide formulations to dry powdered formulations is one way to increase the stability of pharmaceutical peptide formulations. For example, peptide formulations can be dried using various techniques, including spray-drying, lyophilization or freeze-drying, desiccation, spray freeze drying, or any combination thereof. The dry powder peptide formulations achieved by such techniques exhibit significantly increased stability over time at ambient or even physiological temperatures.

The present invention is based, in part, on the surprising discovery that a stable peptide formulation (e.g., a stable glucagon rescue formulation) can be readily prepared by first freeze-drying one or more peptides (e.g., a glucagon peptide) from an aqueous solution containing the peptide(s) to a dry peptide powder. The dried peptide molecules will have a defined ionization profile conferred to them during the drying process. This ionization state may be about equal to the ionization state of the peptide in the aqueous solution from which it was dried (i.e., "about equal" refers to when the ionization profile of the peptide dried from an aqueous solution of pH X corresponds to the ionization profile of the peptide in an aqueous solution wherein the difference between the pH of said aqueous solution and pH X is within (or equal to) 1 pH unit). Whether the ionization state of the peptide in the powder is different (i.e., when the ionization profile of the peptide dried from an aqueous solution of pH X corresponds to the ionization profile of the peptide in an aqueous solution where the difference between the pH of said aqueous solution and pH X is greater than 1 pH unit) from the ionization state of the peptide in the aqueous solution in which it was dried may be dependent on the peptide itself, the components of the aqueous solution (e.g., excipients) and/or the drying method used to prepare the peptide powder.

As a non-limiting example not according to the present invention, if the peptide is dried from an aqueous solution containing a non-volatile buffer, the ionization state of the peptide in the powder may be about equal to that of the peptide in the aqueous solution from which it was dried. However, if the peptide is dried from aqueous solutions containing partially volatile or volatile buffers, the ionization state of the peptide in the powder may not be about equal to the ionization state of the peptide in the aqueous solution from which it was dried. Additionally, if the peptide is dried from an unbuffered solution where the pH has been adjusted using a strong acid and/or strong base (e.g. HCl, NaOH) prior to drying down, the ionization state of the peptide in the powder may be different than the ionization of the peptide in the aqueous solution from which it was dried.

Methods for measuring the ionization state of the dry peptide, or of a peptide in an aprotic polar solvent, are known in the field. In one well-known and accepted method, the ionization state of a peptide can be obtained by reconstituting the dried peptide into un-buffered water and measuring the pH of the reconstituted peptide with a pH indicator such as pH paper or a calibrated pH electrode. Alternatively, the ionization state of a peptide can be determined for a peptide that has been reconstituted in the aprotic polar solvent (e.g., DMSO) by adding at least 20% water to the aprotic polar solvent (e.g., DMSO) and measuring the pH with a pH indicator. See, e.g., Baughman and Kreevoy, "Determination of Acidity in 80% Dimethyl Sulfoxide-20% Water," Journal of Physical Chemistry, 78(4):421-23 (1974). Measurement of pH in an aprotic polar solvent-water solution may require a small correction (i.e., no more than 0.2 pH unit as per Baughman and Kreevoy, supra). Alternatively, spectroscopic techniques can be used to measure the ionization state of a given peptide.

In some embodiments, a dried peptide has an ionization profile that is about equal to the ionization profile of the peptide in the aqueous solution from which it is dried, when the ionization profile of the peptide when it is reconstituted in an aprotic polar solvent is within one pH unit of the pH of the peptide in the aqueous solution from which it is dried (thus, for example, a peptide dried from an aqueous solution of pH 3.0 is said to have an ionization profile about equal to the ionization profile of the peptide in the aqueous solution from which it was dried if the ionization profile of the peptide, when measured in the powder following drying or following reconstitution of said powder in the aprotic polar solvent corresponds to an ionization profile of the peptide in an aqueous solution with pH between 2.0 to 4.0). In some embodiments, a dried peptide has an ionization profile that is not about equal to (or is significantly different from) the ionization profile of the peptide in the aqueous solution from which it is dried when the ionization profile of the peptide when it is reconstituted in an aprotic polar solvent is not within one pH unit of the pH of the peptide in the aqueous solution from which it is dried (thus, for example, a peptide dried from an aqueous solution of pH 3.0 is said to have an ionization profile that is not about equal to (or is significantly different from) the ionization profile of the peptide in the aqueous solution from which it is dried if the ionization profile of the peptide, when measured in the powder following drying or following reconstitution of said powder in the aprotic polar solvent does not correspond to an ionization profile of the peptide in an aqueous solution with pH between 2.0 to 4.0).

In some embodiments, a dried peptide has an ionization profile that is about equal to the ionization profile of the peptide in the aqueous solution from which it is dried, when the ionization profile of the peptide when it is reconstituted in an aprotic polar solvent is within half of a pH unit of the pH of the peptide in the aqueous solution from which it is dried (thus, for example, a peptide dried from an aqueous solution of pH 3.0 is said to have an ionization profile about equal to the ionization profile of the peptide in the aqueous solution from which it was dried if the ionization profile of the peptide, when measured in the powder following drying or following reconstitution of said powder in the aprotic polar solvent corresponds to an ionization profile of the peptide in an aqueous solution with pH between 2.5 to 3.5). In some embodiments, a dried peptide has an ionization profile that is not about equal to (or significantly different from) the ionization profile of the peptide in the aqueous solution from which it is dried when the ionization profile of the peptide when it is reconstituted in an aprotic polar solvent is not within half of a pH unit of the pH of the peptide in the aqueous solution from which it is dried (thus, for example, a peptide dried from an aqueous solution of pH 3.0 is said to have an ionization profile that is not about equal to (or significantly different from) the ionization profile of the peptide in the aqueous solution from which it is dried if the ionization profile of the peptide, when measured in the powder following drying or following reconstitution of said powder in the aprotic polar solvent does not correspond to an ionization profile of the peptide in an aqueous solution with pH between 2.5 to 3.5).

In some disclosures, a dried peptide has an ionization profile that is not about equal to the ionization profile of the peptide in the aqueous solution from which it is dried, when the ionization profile of the peptide when it is reconstituted in an aprotic polar solvent is not within one pH unit of the pH of the peptide in the aqueous solution from which it is dried. In one non-limiting example, an aqueous solution of the peptide glucagon, buffered with a volatile buffer and with a pH adjusted from about 4 to about 5 is dried to a powder wherein the ionization profile of the glucagon peptide in the powder is about equal to the ionization profile of the glucagon peptide in an aqueous solution wherein the pH of said aqueous solution is about 2.0 to about 3.0 to ensure optimal stability, maximal solubility and minimal degradation. When the dried glucagon peptide powder is reconstituted in an aprotic polar solvent (e.g. DMSO), the ionization profile of the glucagon peptide in the aprotic polar solvent is about equal to the ionization profile of the glucagon peptide in the powder.

Once dried, the resulting peptide powder, e.g., the freeze-dried glucagon, is dissolved in an aprotic polar solvent, thereby forming a stable formulation, wherein the moisture or water content of the formulation is less than 15%, 10%, 5%, 1%, 0.5%, 0.25%, 0.15%, or less than 0.1%. The dried peptide maintains its defined ionization profile when reconstituted in the aprotic polar solvent, i.e., the ionization profile of the peptide when reconstituted in the aprotic polar solvent is about equal to the ionization profile the dried peptide. Advantageously, once prepared, the formulation (e.g., the glucagon formulation) is stable for extended periods of time, is ready for use without the need for reconstitution, and is functional over a range of temperatures.

Notably, the formulation technology is a formulation platform technology formulation platform technology is widely applicable for the delivery of numerous other peptides that, like glucagon, have poor or limited stability and solubility in an aqueous environment. The formulation of peptides with an aprotic polar solvent (e.g., DMSO, NMP, ethyl acetate, or a mixture thereof) into high concentration, non-aqueous solutions is an invaluable delivery platform for an important class of therapeutic agentstherapeutic peptides. The stable formulations described herein advantageously promote uniform delivery of the peptide drugs and provide additional shelf stability against aggregation, oxidation, and hydrolysis related degradation pathways.

In certain preferred embodiments, the stable formulations described herein preserve the peptide drugs in a stable form for a prolonged period of time, e.g., for a period of time sufficient to provide a desired shelf life of the formulation without unacceptable levels of physical and chemical degradation of the therapeutic agent prior to use. A desired property of the injectable formulations is that they be non-aqueous and non-reactive with respect to the peptide. In such embodiments, it is possible to store the injectable formulations directly in the injection device itself.

The stable injectable formulations of the present invention contain the desired delivered dose of therapeutic peptide or peptides (e.g., the dose required for drug therapy) and are preferably low volume. For example, in some embodiments an injectable formulation comprising a therapeutic dose of a peptide (e.g., glucagon) has a volume of at least about 1.0 microliters (the lower limit being a function of the filling equipment), more preferably from about 10 milliliters to about 250 microliters. The delivery of a therapeutic dose of peptide at a low volume is accomplished in certain preferred embodiments by concentrating the dose of the therapeutic peptide or peptides (e.g., glucagon) in a stable form in a suitable aprotic polar solvent for injection in accordance with the invention.

Furthermore, the stable formulations of the present invention are suitable for administration without requiring dilution prior to injection. Many currently available therapeutic peptide and vaccine products are produced in a solid particulate form to promote stability while on the shelf. These formulations are diluted prior to injection in sterile water, phosphate buffer solution, or isotonic saline. In contrast, in certain preferred embodiments of the present invention, the therapeutic peptide is concentrated using the particle preparation processing techniques (e.g., spray drying, lyophilization, etc.) routinely employed by the pharmaceutical industry to prepare formulations for injection. In preferred embodiments, therapeutic dosages of peptide drugs are achieved by dissolving the peptides, which have first been freeze-dried (and optionally additional components such as a stabilizing excipient) to a dried powder having very little residual moisture content. Once prepared, the dried peptide powder is dissolved in an aprotic polar solvent, such as DMSO, NMP, ethyl acetate, or blends of these solvents. Thus, in accordance with the goals of the present invention, the low volume, stable formulations of the present invention are injected, infused, or otherwise administered into an animal (e.g., human patient), without first diluting the formulation prior to injection as required by most reconstitution products. As such, in preferred embodiments, the low volume formulations of the present invention are administrable without being first being diluted, or reconstituted, or refrigerated.

### II. Reconstitution of Dried Peptides

In the stable formulations of the present invention, once the peptide (and optional components) are dried to a powder, or where the formulation comprises two or more peptides, once each of the peptides (each optionally also comprising optional component) is dried to a powder, the dried peptide powder is, or the dried peptide powders are, dissolved or reconstituted in an aprotic polar solvent. In some embodiments, the aprotic polar solvent is selected from dimethylsulfoxide (DMSO), dimethylformamide (DMF), ethyl acetate, n-methyl pyrrolidone (NMP), dimethylacetamide (DMA), propylene carbonate, and mixtures thereof. In some embodiments, the aprotic polar solvent is a mixture of two or more of dimethylsulfoxide (DMSO), dimethylformamide (DMF), ethyl acetate, n-methyl pyrrolidone (NMP), dimethylacetamide (DMA), and propylene carbonate. Dimethylsulfoxide (DMSO), ethyl acetate, and n-methyl pyrrolidone (NMP) are particularly preferred aprotic polar solvents, each of which is a biocompatible solvent. In some embodiments, the aprotic polar solvent is dimethylsulfoxide (DMSO). In other embodiments, the aprotic polar solvent is n-methyl pyrrolidone (NMP). In other embodiments, the aprotic polar solvent is a mixture of dimethylsulfoxide (DMSO) and n-methyl pyrrolidone (NMP). In still other embodiments, the aprotic polar solvent is a mixture of dimethylsulfoxide (DMSO) and ethyl acetate. In some embodiments, the dried peptide powder is reconstituted in an aprotic polar solvent that is "neat," i.e., that does not contain a co-solvent. In some embodiments, the dried peptide powder is reconstituted in a solution that comprises an aprotic polar solvent and that does not contain water as a co-solvent.

In some embodiments, the formulations of the present invention further comprise at least one co-solvent that depresses the freezing point of the formulation. The co-solvent is a polar protic solvent. In some embodiment, the co-solvent is selected from ethanol, propylene glycol (PG), glycerol, and mixtures thereof. In some embodiments, the co-solvent is ethanol or propylene glycol (PG). The co-solvent may be present in the formulation in an amount ranging from about 10% (w/v) to about 50% (w/v), e.g., about 10%, about 15%, about 20%, about 25%, about 30%, about 35%, about 40%, about 45%, or about 50% (w/v). In some embodiments, the co-solvent is present in the formulation in an amount ranging from about 10% (w/v) to about 50% (w/v), from about 10% (w/v) to about 40% (w/v), from about 10% (w/v) to about 30% (w/v), from about 10% (w/v) to about 25% (w/v), from about 15% (w/v) to about 50% (w/v), from about 15% (w/v) to about 40% (w/v), from about 15% (w/v) to about 30% (w/v), or from about 15% (w/v) to about 25% (w/v). In some embodiments, the at least one co-solvent depresses the freezing point of the formulation by at least 5 °C., at least 10 °C, at least 15 °C, at least 20 °C or more as compared to an otherwise identical formulation that does not comprise the co-solvent. In some embodiments, the at least one co-solvent depresses the freezing point of the formulation to about 3 °C, to about 2 °C, to about 1 °C, or to about 0 °C or below.

### III. Stabilizing Excipients

In certain preferred embodiments, the formulations described herein may be further stabilized to ensure the stability of the peptide incorporated therein. In some embodiments, the stability of the injectable formulation is enhanced by the inclusion of one or more stabilizing agents or stabilizing excipients into the formulation prior to the drying of the peptide or peptides. In other embodiments, the stability of the injectable formulation is enhanced by reconstituting the dried peptide or peptides with a stabilizing agent or stabilizing excipient in the aprotic polar solvent.

In some embodiments, the stabilizing excipient is a cryoprotectant. The addition of a cryoprotectant, such as trehalose, protects the peptide formulations of the present invention against instability associated with freeze-thaw cycles. The addition of the cryoprotectant trehalose also promotes enhanced thawing of a frozen peptide formulation. This property of enhanced thawing is surprisingly advantageous, particularly in emergency medical situations, such as a severe hypoglycemia episode, wherein a peptide formulation of the present invention is frozen and needs to be administered quickly. Thus, in another aspect of the present invention, the stable formulation has an improved freeze-thaw stability, an enhanced thawing rate, and/or an enhanced thawing profile.

In some embodiments, the stabilizing excipient is selected from sugars, starches, sugar alcohols, and mixtures thereof. Examples of suitable sugars for stabilizing excipients include, but are not limited to, trehalose, glucose, sucrose, etc. Examples of suitable starches for stabilizing excipients include, but are not limited to, hydroxyethyl starch (HES). Examples of suitable sugar alcohols for stabilizing excipients include, but are not limited to, mannitol and sorbitol. In some embodiments, the at least one stabilizing excipient (e.g., a sugar, a starch, a sugar alcohol, or a mixture thereof) is capable of enhancing the stability of the peptide during a freeze-thawing process, enhancing the thawing rate of the formulation, or enhancing the thawing profile of the formulation.

In some embodiments, the stabilizing excipient is present in the formulation in an amount ranging from about 1% (w/v) to about 60% (w/v), from about 1% (w/v) to about 50% (w/v), from about 1% (w/v) to about 40% (w/v), from about 1% (w/v) to about 30% (w/v), from about 1% (w/v) to about 20% (w/v), from about 5% (w/v) to about 60% (w/v), from about 5% (w/v) to about 50% (w/v), from about 5% (w/v) to about 40% (w/v), from about 5% (w/v) to about 30% (w/v), from about 5% (w/v) to about 20% (w/v), from about 10% (w/v) to about 60% (w/v), from about 10% (w/v) to about 50% (w/v), from about 10% (w/v) to about 40% (w/v), from about 10% (w/v) to about 30% (w/v), or from about 10% (w/v) to about 20% (w/v). In some embodiments, the stabilizing excipient is present in the formulation in an amount that is about 1%, about 5%, about 10%, about 15%, about 20%, about 25%, about 30%, about 35%, about 40%, about 45%, about 50%, about 55%, or about 60% (w/v).

In formulations comprising two or more peptides, in some embodiments each of the peptides are dried in a mixture comprising a stabilizing excipient. The mixtures of the peptide and the stabilizing excipient may be the same for each peptide, or the stabilizing excipient that is used for drying each peptide may be different. In other embodiments, some but not all of the peptides may be dried in a mixture comprising a stabilizing excipient, while other peptides may be dried from an aqueous solution in the absence of a stabilizing excipient.

In some embodiments, the formulation further comprises additional stabilizing agents including, for example, antioxidants, chelators and preservatives. Examples of suitable antioxidants include, but are not limited to, ascorbic acid, cysteine, methionine, monothioglycerol, sodium thiosulphate, sulfites, BHT, BHA, ascorbyl palmitate, propyl gallate, N-acetyl-L-cysteine (NAC), and Vitamin E. Examples of suitable chelators include, but are not limited to, EDTA, tartaric acid and salts thereof, glycerin, and citric acid and salts thereof. Examples of suitable preservatives include, but are not limited to, benzyl alcohols, methyl parabens, propyl parabens, and mixtures thereof.

In some embodiments, the formulation further comprises a stabilizing polyol. Such formulations and materials are described, for example, in U.S. Pat. Nos. 6,290,991 and 6,331,310.

### IV. Methods of Making Stable Peptide Formulations

In yet another aspect, the present invention provides a process according to claim 14 for making a stable formulation for parenteral injection, the process comprising: drying a peptide from an aqueous peptide solution to a dry peptide powder wherein the peptide in the powder has an ionization profile about equal to the ionization profile of the peptide in an aqueous solution wherein the pH of said aqueous solution is about equal to a pH of optimal stability and solubility for the peptide; and reconstituting the dry peptide powder with an aprotic polar solvent, thereby making the stable formulation. In some embodiments, the dried peptide powder has an ionization profile that is about equal to the ionization profile of the peptide in the aqueous solution from which it was dried, and the peptide maintains the ionization profile that is about equal to the ionization profile of the peptide in the aqueous solution from which it was dried when the dried peptide powder is reconstituted in the aprotic polar solvent. In other disclosures the dried peptide powder has an ionization profile that is not equal to the ionization profile of the peptide in the aqueous solution from which it was dried, and the peptide maintains the ionization profile when the dried peptide powder is reconstituted in the aprotic polar solvent.

The process for making stable peptide formulations is a platform formulation process that may be used to formulate any peptide that has limited or poor stability or solubility in an aqueous environment. Peptides (or salts thereof) suitable for use in the formulations not according to the present invention include, but are not limited to, insulin, leuprolide, an luteinizing-hormone-releasing hormone (LHRH) agonists, pramlintide, parathyroid hormone (PTH), amylin, botulinum toxin, a conotoxin, hematide, an amyloid peptide, cholecystikinin, gastric inhibitory peptide, an insulin-like growth factor, growth hormone releasing factor, anti-microbial factor, glatiramer, glucagon-like peptide-1 (GLP-1), a GLP-1 agonist, exenatide, and analogs thereof. The peptide of the invention is glucagon.

Still further, the technology can be applied to all types of peptides irrespective of the underlying disease state. By way of example, the stable formulations of the present invention can include one, two, three, four, or more peptides or salts, analogs, and/or mixtures thereof. Peptides (as well as salts thereof) suitable for use in the formulations using the underlying concept of the present invention include, but are not limited to, glucagon,
pramlintide, insulin, leuprolide, an luteinizing-hormone-releasing hormone (LHRH) agonist, parathyroid hormone (PTH), amylin, botulinum toxin, hematide, an amyloid peptide, cholecystikinin, gastric inhibitory peptide, an insulin-like growth factor, growth hormone releasing factor, anti-microbial factor, glatiramer, glucagon-like peptide-1 (GLP-1), a GLP-1 agonist, exenatide, analogs thereof, and mixtures thereof. In some embodiments, the peptide is a hydrochloride salt or an acetate salt. Again, peptides other than those listed above are contemplated as also being useful.

In some embodiments, two, three, four or more peptides are formulated into a stable formulation. In embodiments where two or more peptides are formulated into the stable formulation, each peptide is separately dried from an aqueous solution to a dry peptide powder, and each dried peptide powder has an ionization profile about equal to the ionization profile of the peptide in an aqueous solution wherein the pH of said aqueous solution is about equal to a pH of optimal stability and solubility for the peptide. The two or more dried peptide powders are reconstituted with an aprotic polar solvent, and wherein each dried peptide powder maintains the ionization state of the peptide that is about equal to the ionization state of the peptide in an aqueous solution wherein the pH of said aqueous solution is about equal to a pH of optimal stability and solubility for that peptide (i.e., the first dried peptide maintains the first ionization profile when the first dried peptide is reconstituted in the aprotic polar solvent, and the second dried peptide maintains the second ionization profile when the second dried peptide is reconstituted in the aprotic polar solvent).

As explained above, when the peptide is solubilized in the aqueous solution, the pH of the aqueous solution is adjusted such that the dried peptide has an ionization profile that is about equal to the ionization profile of the peptide in an aqueous solution wherein the pH of said aqueous solution is about equal to a pH of optimal stability and solubility for the peptide. Once dried, the peptide will have an ionization profile of optimal stability/minimal degradation and will retain that ionization profile when dissolved in or reconstituted in the aprotic polar solvent. As such, in one embodiment, the ionization state of the peptide in the formulation should be about equal to the ionization state of the peptide in an aqueous solution wherein the pH of said aqueous solution is about 2 to about 3. In another embodiment, the ionization state of the peptide in the formulation should be about equal to the ionization state of the peptide in an aqueous solution wherein the pH of said aqueous solution is about 4 to about 6. In yet another embodiment, the ionization state of the peptide in the formulation should be about equal to the ionization state of the peptide in an aqueous solution wherein the pH of said aqueous solution is about 4 to about 5. In yet another embodiment, the ionization state of the peptide in the formulation should be about equal to the ionization state of the peptide in an aqueous solution wherein the pH of said aqueous solution is about 6 to 8, 8 to 10, or 10 to 12.

Once the peptide (and optionally other components, such as a stabilizing excipient, that are added to the aqueous solution containing the peptide before drying) are dried to a powder, the dried peptide powder is dissolved or reconstituted in an aprotic polar solvent as described herein (e.g., dimethylsulfoxide (DMSO), n-methyl pyrrolidone (NMP), ethyl acetate, and mixtures thereof). In some embodiments, the aprotic polar solvent is dimethylsulfoxide (DMSO). In other embodiments, the aprotic polar solvent is n-methyl pyrrolidone (NMP). In further embodiments, the aprotic polar solvent is a mixture of DMSO and NMP.

In some embodiments, the step of reconstituting the dried peptide powder comprises diluting or reconstituting the dried peptide with a mixture comprising an aprotic polar solvent and a co-solvent that depresses the freezing point of the formulation. In some embodiments, the co-solvent is selected from ethanol, propylene glycol, glycerol, and mixtures thereof. In some embodiments, the co-solvent is present in the formulation in an amount ranging from about 10% (w/v) to about 50% (w/v), e.g., about 10%, about 15%, about 20%, about 25%, about 30%, about 35%, about 40%, about 45%, or about 50% (w/v).

In the foregoing process, drying of the peptide compound from the aqueous solution (optionally containing excipients) is carried out using spray-drying techniques, freeze-drying techniques or lyophilization techniques. Spray-drying techniques are well known to those skilled in the art. Spray-drying includes the steps of atomization of a solution containing one or more solutes (e.g., therapeutic agent, excipients) via a nozzle spinning disk, or other device, followed by evaporation of the solvent from the droplets. The nature of the powder that results is the function of several variables processing parameters, including the initial solute concentration, size distribution of droplets produced and the rate of solute removal. The particles produced may comprise aggregates of primary particles which consist of crystals and/or amorphous solids depending on the rate and conditions of solvent removal.

A spray-drying process for preparing ultra-fine powders of biological macromolecules such as proteins, oligo-peptides, high molecular weight polysaccharides, and nucleic acids is described in, for example, U.S. Pat. No. 6,051,256. Freeze-drying procedures are well-known in the art, and are described, for example, in U.S. Pat. No. 4,608,764 and U.S. Pat. No. 4,848,094. Spray-freeze-drying processes are described, e.g., in U.S. Pat. No. 5,208,998. Other spray-drying techniques are described, for example, in U.S. Pat. Nos. 6,253,463; 6,001,336; 5,260,306; and PCT International Publication Nos. WO 91/16882 and WO 96/09814.

Lyophilization techniques are well-known to those skilled in the art. Lyophilization is a dehydration technique that takes place while a product is in a frozen state (ice sublimation under a vacuum) and under a vacuum (drying by gentle heating). These conditions stabilize the product, and minimize oxidation and other degradative processes. The conditions of freeze drying permit running the process at low temperatures, therefore thermally labile products can be preserved. Steps in freeze drying include pre-treatment, freezing, primary drying and secondary drying. Pre-treatment includes any method of treating the product prior to freezing. This may include concentrating the product, formulation revision (i.e., addition of components to increase stability and/or improve processing), decreasing a high vapor pressure solvent or increasing the surface area. Methods of pre-treatment include: freeze concentration, solution phase concentration, and formulating specifically to preserve product appearance or to provide lyoprotection for reactive products, and are described, e.g., in U.S. Pat. No. 6,199,297. "Standard" lyophilization conditions, are described, e.g., in U.S. Pat. No. 5,031,336, and in "Freeze Drying of Pharmaceuticals" (DeLuca, Patrick P., J. Vac. Sci. Technol., Vol. 14, No. 1, January/February 1977); and "The Lyophilization of Pharmaceuticals: A Literature Review" (Williams, N. A., and G. P. Polli, Journal of Parenteral Science and Technology, Vol. 38, No. 2, March/April 1984).

In certain preferred embodiments, the lyophilization cycle is partially performed above the glass transition temperature (T_{g}) of the therapeutic agent formulation to induce a collapse of the mass to form a dense cake containing residue moisture. In other embodiments, the lyophilization cycle is carried out below the glass transition temperature in order to avoid a collapse in order to achieve a complete drying of the particles.

### V. Therapeutic Methods

In another aspect, the present invention provides a formulation for use in methods of treating diseases or conditions by administering to a subject a stable formulation as described herein in an amount effective to treat, alleviate or prevent the disease, condition or disorder. In some embodiments, the disease, condition, or disorder to be treated with a stable formulation of the present invention is a diabetic condition. Examples of diabetic conditions include, but are not limited to, type 1 diabetes, type 2 diabetes, gestational diabetes, pre-diabetes, hyperglycemia, hypoglycemia, and metabolic syndrome. In some embodiments, the disease, condition, or disorder is hypoglycemia. In some embodiments, the disease, condition, or disorder is diabetes.

In some embodiments, the formulation for use in a therapeutic method of the present invention comprises treating hypoglycemia by administering to a subject having hypoglycemia a stable formulation as described herein in an amount effective to treat the hypoglycemia. The subject is administered a stable formulation comprising glucagon.

In some embodiments, the formulation for use in a therapeutic method of the present invention comprises treating diabetes by administering to a subject having diabetes a stable formulation as described herein in an amount effective to treat the diabetes. In some embodiments, the subject is administered a stable formulation comprising glucagon and exenatide.

Administered dosages for the peptide drugs as described herein for treating a disease, condition, disorder (e.g., a diabetic condition, e.g., hypoglycemia or diabetes) are in accordance with dosages and scheduling regimens practiced by those of skill in the art. General guidance for appropriate dosages of all pharmacological agents used in the present methods is provided in Goodman and Gilman's The Pharmacological Basis of Therapeutics, 11 th Edition, 2006, supra, and in a Physicians' Desk Reference (PDR), for example, in the 65th (2011) or 66th (2012) Eds., PDR Network, LLC. The appropriate dosage of a peptide drug for treating a disease, condition, or disorder as described herein will vary according to several factors, including the formulation of the composition, patient response, the severity of the condition, the subject's weight, and the judgment of the prescribing physician. Effective doses of the described formulations deliver a medically effective amount of a peptide drug. The dosage can be increased or decreased over time, as required by an individual patient.

Determination of an effective amount or dose is well within the capability of those skilled in the art, especially in light of the detailed disclosure provided herein. Generally, the formulations to deliver these doses may contain one, two, three, four, or more peptides, wherein each peptide is present at a concentration from about 0.1 mg/mL up to the solubility limit of the peptide in the formulation. This concentration is preferably from about 1 mg/mL to about 100 mg/mL, e.g., about 1 mg/mL, about 5 mg/mL, about 10 mg/mL, about 15 mg/mL, about 20 mg/mL, about 25 mg/mL, about 30 mg/mL, about 35 mg/mL, about 40 mg/mL, about 45 mg/mL, about 50 mg/mL, about 55 mg/mL, about 60 mg/mL, about 65 mg/mL, about 70 mg/mL, about 75 mg/mL, about 80 mg/mL, about 85 mg/mL, about 90 mg/mL, about 95 mg/mL, or about 100 mg/mL.

The formulations of the present invention may be for subcutaneous, intradermal, or intramuscular injection. In some embodiments, the formulation is administered subcutaneously.

The formulations of the present disclosure can be administered by injection using any suitable device. For example, a formulation of the present invention may be placed into a syringe, a pen injection device, an auto-injector device, or a pump device. In some embodiments, the injection device is a multi-dose injector pump device or a multi-dose auto-injector device. The formulation is presented in the device in such a fashion that the formulation is readily able to flow out of the needle upon actuation of an injection device, such as an auto-injector, in order to deliver the peptide drugs. Suitable pen/auto injector devices include, but are not limited to, those pen/auto injection devices manufactured by Becton-Dickenson, Swedish Healthcare Limited (SHL Group), YpsoMed Ag, and the like. Suitable pump devices include, but are not limited to, those pump devices manufactured by Tandem Diabetes Care, Inc., Delsys Pharmaceuticals and the like.

In some embodiments, the formulations of the present invention are provided ready for administration in a vial, a cartridge, or a pre-filled syringe.

In another aspect, the present invention provides for the use of a stable formulation as described herein for the formulation of a medicament for the treatment of any disease, condition, or disorder that may be treated with the peptide of the formulation. In some embodiments, the stable formulation is used for formulating a medicament for the treatment of a diabetic condition, e.g., type 1 diabetes, type 2 diabetes, gestational diabetes, pre-diabetes, hyperglycemia, hypoglycemia, or metabolic syndrome.

In some embodiments, the stable formulation is used for formulating a medicament for the treatment of hypoglycemia. The stable formulation comprises glucagon or a salt thereof (e.g., glucagon acetate). In some embodiments, the stable formulation comprises glucagon and exenatide.

In some embodiments, the stable formulation is used for formulating a medicament for the treatment of diabetes.

### VI. Kits

The present disclosure provides kits for treating a disease, condition or disorder as described herein. In some embodiments, the kit comprises: a stable formulation comprising one, two, three, four or more peptides or salts thereof, wherein the peptide(s) has been dried in a non-volatile buffer, and wherein the dried peptide(s) has a pH memory that is about equal to the pH of the peptide(s) in the non-volatile buffer; and an aprotic polar solvent; wherein the moisture content of the formulation is less than 5%, and wherein the dried peptide(s) maintains the pH memory that is about equal to the pH of the peptide(s) in the non-volatile buffer when the dried peptide(s) is reconstituted in the aprotic polar solvent; and a syringe for administration of the stable formulation to the subject.

In some disclosures, the kit comprises a stable glucagon formulation as described herein for use in treating hypoglycemia in a subject. In some disclosures, the kit comprises a glucagon formulation comprising: glucagon or a salt thereof (e.g., glucagon acetate), wherein the glucagon has been dried in a non-volatile buffer, and wherein the dried glucagon has a pH memory that is about equal to the pH of the glucagon in the non-volatile buffer selected from a glycine buffer, a citrate buffer, a phosphate buffer, and mixtures thereof, wherein the pH memory of the dried glucagon is from about 2.0 to about 3.0; and an aprotic polar solvent selected from dimethylsulfoxide (DMSO), n-methyl pyrrolidone (NMP), ethyl acetate, and mixtures thereof; wherein the moisture content of the formulation is less than 1%, and wherein the dried glucagon maintains the pH memory that is about equal to the pH of the glucagon in the non-volatile buffer when the dried glucagon is reconstituted in the aprotic polar solvent. In some embodiments, the glucagon formulation further comprises a co-solvent that depresses the freezing point of the formulation, wherein the co-solvent is selected from ethanol, propylene glycol, glycerol, and mixtures thereof. In some embodiments, the glucagon formulation further comprises a stabilizing excipient selected from sugars, starches, and mixtures thereof. In some embodiments, the glucagon is present in the formulation in an amount ranging from about 1 mg/mL to about 50 mg/mL.

In some disclosures, the kit comprises a stable insulin and pramlintide formulation as described herein for use in treating diabetes in a subject. In some disclosures, the kit comprises an insulin and pramlintide formulation comprising: insulin, wherein the insulin has been dried in a first non-volatile buffer selected from a glycine buffer, a citrate buffer, a phosphate buffer, and mixtures thereof, and wherein the dried insulin has a first pH memory that is about equal to the pH of the insulin in the first non-volatile buffer, wherein the first pH memory is from about 1.5 to about 2.5 or from about 6.0 to about 8.0; pramlintide, wherein the pramlintide has been dried in a second non-volatile buffer selected from a glycine buffer, a citrate buffer, a phosphate buffer, and mixtures thereof, and wherein the dried pramlintide has a second pH memory that is about equal to the pH of the pramlintide in the second non-volatile buffer, wherein the second pH memory is from about 3.0 to about 5.0 or from about 4.0 to about 6.0; and an aprotic polar solvent selected from dimethylsulfoxide (DMSO), n-methyl pyrrolidone (NMP), ethyl acetate, and mixtures thereof; wherein the moisture content of the formulation is less than 1%, wherein the dried insulin maintains the first pH memory that is about equal to the pH of the insulin in the first non-volatile buffer when the dried insulin is reconstituted in the aprotic polar solvent, and wherein the dried pramlintide maintains the second pH memory that is about equal to the pH of the pramlintide in the second non-volatile buffer when the dried pramlintide is reconstituted in the aprotic polar solvent. In some disclosures, the insulin and pramlintide formulation further comprises a co-solvent that depresses the freezing point of the formulation, wherein the co-solvent is selected from ethanol, propylene glycol, glycerol, and mixtures thereof. In some disclosures, one or both of the insulin in the first non-volatile buffer and the pramlintide in the second non-volatile buffer further comprises a stabilizing excipient selected from sugars, starches, and mixtures thereof. In some disclosures, the first non-volatile buffer and the second non-volatile buffer are the same. In some disclosures, the first non-volatile buffer and the second non-volatile buffer are different. In some disclosures, each of the insulin and pramlintide is present in the formulation in an amount ranging from about 1 mg/mL to about 50 mg/mL. In some disclosures, the first pH memory is from about 1.5 to about 2.5. In some embodiments, the first pH memory is from about 6.0 to about 8.0. In some embodiments, the second pH memory is from about 3.0 to about 5.0. In some disclosures, the second pH memory is from about 4.0 to about 6.0. In some disclosures, the first pH memory is from about 1.5 to about 2.5 and the second pH memory is from about 3.0 to about 5.0.

In some disclosures, the kit comprises a syringe that is part of a pen injection device, an auto-injector device or a pump. In some disclosures, the syringe is prefilled with the stable formulation. In some disclosures, the kit further comprises instructions, wherein the instructions direct the administration of the stable formulation to treat the subject in need thereof (e.g., the subject having hypoglycemia or diabetes).

In another instance, the kit can include: (a) a first composition comprising an organic solvent system that includes an organic solvent, an organic phase buffer, and a peptide or salt thereof having a first ionization profile that corresponds to the peptide's optimal stability and solubility; and (b) a second composition comprising an aprotic polar solvent capable of partially or fully solubilizing the peptide or salt thereof and maintaining the first ionization profile. The first and second compositions can be included in first and second containers, respectively. Alternatively, the kit can be designed such that both compositions are in a single container but are separated by a separating piece that can be removed or otherwise modified as desired to allow both compositions to come into contact with one another. The kit can include instructions to mix the contents of both containers or to break or remove the separator piece so as to obtain a two-phased system in which one of the phases includes the aprotic polar solvent in which the peptide or salt there is solubilized in said solvent. The other phase can include the organic solvent system. The kit can include an apparatus to separate the two phases and to inject the aprotic polar solvent having the peptide or salt thereof into a patient.

### EXAMPLES

The following examples are offered for illustrative purposes, and are not intended to limit the invention in any manner. Those of skill in the art will readily recognize a variety of noncritical parameters which can be changed or modified to yield essentially the same results.

### Illustrating Example 1 (demonstrating the principle underlying the invention using pramlintide as model peptide) (Peptide pH Memory in Acetate Buffers)

Pramlintide acetate (a 37-amino acid, synthetic analogue of the human hormone amylin) was dissolved in an aqueous solution (pH 4.0) containing 5% (w/v) trehalose and buffered with 10 mM acetate. Following addition of the pramlintide salt, the pH of the solution was re-adjusted to 4.0 with 1.0 N HCl. The final concentration of pramlintide acetate in the aqueous solution was 0.9 mg/mL. The solution was dried down to a powder via lyophilization. Following drying, the pH of the peptide powder was assessed by reconstituting to the original (pre-lyo) concentration (0.9 mg/mL) with unbuffered water. The pH of the reconstituted samples (N = 3) were found to be between 4.1 and 4.2.

Sample solutions (N = 3) were prepared by solubilizing the lyophilized powder directly in neat DMSO to a concentration of 0.9 mg/mL pramlintide acetate. To test formulation stability in the polar aprotic solvent, the samples (N = 3) were stored in sealed glass vials at 40 °C for six weeks. Following six weeks of storage, the sample solutions (N = 3) were clear and absent of precipitation and visible aggregates; the remaining pramlintide concentration at T = 6 weeks was found to be 99.6% of the initial measurement (T = 0 weeks) as assessed by RP-HPLC.

### Illustrating Example 2 (demonstrating the principle underlying the invention) (Prophetic Examples)

In addition to the pramlintide acetate formulations prepared in Illustrating Example 1, formulations of the present invention can be prepared in a variety of ways. FIG. 1 provides non-limiting examples of two such processes. The first inventive process, which utilizes an aqueous composition, can include the following steps:
Step 1: Select a therapeutic agent, such as a peptide or salt thereof. Determine its isoelectric point and the pH in which an optimal ionization state is imparted to the agent so that it has optimal stability and solubility in an aqueous solution.
Step 2: Add the therapeutic agent to an aqueous solution that includes a component that imparts a desired pH in said solution. Said component includes a strong acid in amounts sufficient to establish the desired pH. The component can also include a non-volatile buffer. The pH of the aqueous solution can be about equal (i.e., within 1 pH unit) to the pH of optimal stability and solubility for the therapeutic agent. Alternatively, the pH of the aqueous solution can be at least 1 pH unit greater than the pH of optimal stability and solubility for the therapeutic agent. In either instance, the aqueous solution can be designed such that the pH remains relatively constant upon drying or such that the pH of the solution changes to fall within the optimal pH upon drying.
Step 3: Subject the aqueous solution to a drying step (e.g., lyophilization or spray drying) to produce a dried or powdered form of said therapeutic agent. The dried or powdered form of said therapeutic agent will have an ionization profile that corresponds to the agent's optimal stability and solubility in an aqueous solution.
Step 4: Reconstitute the dried therapeutic agent or powder in an aprotic polar solvent such that the reconstituted agent will also have an ionization profile that is about equal to the ionization profile of the dried agent. Notably, this result can be obtained irrespective of whether the initial pH in the aqueous solution in step 2 is within or outside the optimal pH for the therapeutic agent.

The second comparative process, which utilizes a non-aqueous composition, can include the following steps:
Step 1: Select a therapeutic agent, such as a peptide or salt thereof. Determine its isoelectric point and the pH in which an optimal ionization state is imparted to the agent so that it has optimal stability and solubility in an aqueous solution. The dried therapeutic agent from step 3 in process 1 above can be used if desired. However, the agent does not have to be dried or does not have to be dried in the manner described above in step 3 for this process (see FIG. 1).
Step 2: Add the therapeutic agent to an organic solvent system that includes an organic solvent and an organic phase buffer. The pH of the organic solvent system can be about equal (i.e., within 1 pH unit) to the pH of optimal stability and solubility for the therapeutic agent, as measured by placing said agent in an aqueous solution. In preferred aspects, the therapeutic agent is suspended or partially suspended in the organic solvent system.
Step 3: Add an aprotic polar solvent into the organic solvent system in an amount sufficient such that a two-phased system is obtained via solvent exchange. The first phase includes the polar aprotic solvent and at least a portion of the therapeutic agent in a solubilized form and the second phase includes the organic solvent system having the organic solvent and the organic buffer. The organic solvent system is substantially anhydrous. The organic solvent system can be removed from the polar aprotic solvent by separation methods known in the art.

Additionally, co-formulations can be prepared such that a single formulation includes at least two different peptides or at least three different peptides or at least four different peptides or at least five different peptides or more. Each of the additional peptides can be prepared by either of the two processes discussed above.

## Claims

1. A stable formulation for parenteral injection comprising:
(a) a peptide or a salt thereof that has been previously dried from an aqueous composition comprising a strong acid, wherein the dried peptide or salt thereof has a first ionization profile that corresponds to the peptide's optimal stability and solubility; and
(b) an aprotic polar solvent, wherein the dried peptide or salt thereof is reconstituted into an aprotic polar solvent and has a second ionization profile in the aprotic polar solvent,
wherein the first and second ionization profiles are within 1 pH unit of one another,
wherein the aqueous composition does not include a non-volatile buffer, and
wherein the peptide or salt thereof is glucagon.

2. The stable formulation of claim 1, wherein the dried peptide is partially or fully solubilized within the aprotic polar solvent, preferably fully solubilized in said aprotic polar solvent.

3. The stable formulation of any one of claims 1 to 2, wherein the aqueous composition comprises hydrochloric acid.

4. The stable formulation of claim 3, wherein the aqueous composition does not include a buffer.

5. The stable formulation of any one of claims 1 to 4, wherein the drying is performed by lyophilization, spray drying, desiccation, thin-film freezing, spray freeze drying, or any combination thereof.

6. The stable formulation of any one of claims 1 to 5, wherein the moisture content of the formulation is less than 15%, or less than 10%, or less than 5%, or less than 1%, or is anhydrous.

7. The stable formulation of any one of claims 1 to 6, wherein the aprotic polar solvent is selected from dimethylsulfoxide (DMSO), n-methyl pyrrolidone (NMP), ethyl acetate, dimethylformamide (DMF), propylene carbonate, or mixtures thereof.

8. The stable formulation of any one of claims 1 to 7, further comprising a co-solvent that depresses the freezing point of the formulation, wherein the co-solvent is selected from ethanol, propylene glycol, glycerol, and mixtures thereof.

9. The stable formulation of any one of claims 1 to 8, further comprising a stabilizing excipient selected from a sugar, a starch, and mixtures thereof.

10. The stable formulation of claim 9, wherein the stabilizing excipient is trehalose.

11. The stable formulation of any one of claims 9 to 10, wherein the stabilizing excipient is present in the formulation in an amount ranging from 1% (w/v) to 20% (w/v).

12. The stable formulation of any one of claims 1 to 11, wherein the first or second or both ionization profiles correspond to the ionization profile of glucagon when solubilized in an aqueous solution having a pH range of 2 to 3.

13. The stable formulation of any one of claims 1 to 12, wherein the stable formulation is comprised in a syringe, a pen injection device, an auto-injector device, or a pump device.

14. A method of making any one of the stable formulations of claims 1 to 13 for parenteral injection, the method comprising:
(a) drying an aqueous composition comprising a peptide or a salt thereof and a strong acid, to a dried peptide powder, wherein the dried peptide powder has a first ionization profile that corresponds to the peptide's optimal stability and solubility; and
(b) reconstituting the dried peptide powder in an aprotic polar solvent, wherein the reconstituted dried peptide powder has a second ionization profile in the aprotic polar solvent,
wherein the first and second ionization profiles are within 1 pH unit of one another,
wherein the peptide or salt thereof is glucagon, and
wherein the aqueous composition has a pH range of greater than 3 to 7 prior to drying and wherein the first or second or both ionization profiles correspond to the ionization profile of glucagon when solubilized in an aqueous solution having a pH range of 2 to 3.

15. The stable formulation of any of claims 1 to 13 or made according to claim 14 for use as a medicament, in particular for use as a medicament for the treatment of a diabetic condition.

## Patentansprüche

1. Stabile Formulierung für parentale Injektion, die
(a) Peptid oder ein Salz davon, das vorher aus einer wässrigen Zusammensetzung, die eine starke Säure umfasst, getrocknet wurde, wobei das getrocknete Peptid oder Salz davon ein erstes Ionisationsprofil aufweist, das der optimalen Stabilität und Löslichkeit des Peptids entspricht, und
(b) aprotisches polares Lösungsmittel, wobei das getrocknete Peptid oder das Salz davon in aprotischem polaren Lösungsmittel wiederhergestellt wird und ein zweites Ionisationsprofil in dem aprotischen polaren Lösungsmittel aufweist,
umfasst,
wobei das erste und zweite Ionisationsprofil innerhalb 1 pH-Einheit voneinander sind,
wobei die wässrige Zusammensetzung keinen nicht-flüchtigen Puffer einschließt, und
wobei das Peptid oder das Salz davon Glucagon ist.

2. Stabile Formulierung nach Anspruch 1, bei der das getrocknete Peptid teilweise oder vollständig in dem aprotischen polaren Lösungsmittel gelöst ist, vorzugsweise vollständig in dem aprotischen polaren Lösungsmittel gelöst ist.

3. Stabile Formulierung nach Anspruch 1 oder 2, bei der die wässrige Zusammensetzung Salzsäure umfasst.

4. Stabile Formulierung nach Anspruch 3, bei der die wässrige Zusammensetzung keinen Puffer einschließt.

5. Stabile Formulierung nach einem der Ansprüche 1 bis 4, bei der der die Trocknung mittels Gefriertrocknung, Sprühtrocknung, Labortrocknung, Dünnfilm-Gefriertrocknung, Sprüh-Gefriertrocknung oder einer Kombination davon durchgeführt wird.

6. Stabile Formulierung nach einem der Ansprüche 1 bis 5, bei der der Feuchtigkeitsgehalt der Formulierung weniger als 15%, oder weniger als 10%, oder weniger als 5%, oder weniger als 1% beträgt oder wasserfrei ist.

7. Stabile Formulierung nach einem der Ansprüche 1 bis 6, bei der das aprotische polare Lösungsmittel ausgewählt ist aus Dimethylsulfoxid (DMSO), N-Methylpyrrolidon (NMP), Ethylacetat, Dimethylformamid (DMF), Propylencarbonat, oder Mischungen davon.

8. Stabile Formulierung nach einem der Ansprüche 1 bis 7, die ferner ein Co-Lösungsmittel umfasst, das den Gefrierpunkt der Formulierung herabsetzt, wobei das Co-Lösungsmittel ausgewählt ist aus Ethanol, Propylenglycol, Glycerin, und Mischungen davon.

9. Stabile Formulierung nach einem der Ansprüche 1 bis 8, das ferner stabilisierendes Hilfsmittel ausgewählt aus Zucker, Stärke und Mischungen davon umfasst.

10. Stabile Formulierung nach Anspruch 9, bei der das stabilisierende Hilfsmittel Trehalose ist.

11. Stabilisierende Formulierung nach einem der Ansprüche 9 bis 10, bei der das stabilisierende Hilfsmittel in der Formulierung in einer Menge von 1% (Gewichtsvolumen) bis 20% (Gewichtsvolumen) vorliegt.

12. Stabile Formulierung nach einem der Ansprüche 1 bis 11, bei der das erste oder zweite oder beide Ionisationsprofile dem Ionisationsprofil von Glucagon entsprechen, das in einer wässrigen Lösung mit einem pH von 2 bis 3 gelöst ist.

13. Stabile Formulierung nach einem der Ansprüche 1 bis 12, bei der die stabile Formulierung in einer Spritze, einer Stift-Injektionsvorrichtung, einer Autoinjektionsvorrichtung oder einer Pumpenvorrichtung umfasst ist.

14. Verfahren zur Herstellung der stabilen Formulierung gemäß einem der Ansprüche 1 bis 13 für parentale Injektion, bei welchem Verfahren
(a) wässrige Zusammensetzung, die Peptid oder ein Salz davon und eine starke Säure umfasst, zu einem getrockneten Peptidpulver getrocknet wird, wobei das getrocknete Peptidpulver ein erstes Ionisationsprofil aufweist, das der optimalen Stabilität und Löslichkeit des Peptids entspricht, und
(b) das getrocknete Peptidpulver in aprotischem polaren Lösungsmittel wiederhergestellt wird, wobei das wiederhergestellte getrocknete Peptidpulver ein zweites Ionisationsprofil in dem aprotischen polaren Lösungsmittel aufweist,
wobei das erste und zweite Ionisationsprofil innerhalb einer pH-Einheit voneinander sind,
wobei das Peptid oder das Salz davon Glucagon ist, und
wobei die wässrige Zusammensetzung einen pH-Bereich von größer als 3 bis 7 vor dem Trocknen aufweist und wobei das erste oder zweite oder beide Ionisationsprofile dem Ionisationsprofil von Glucagon entsprechen, das in einer wässrigen Lösung mit einem pH im Bereich von 2 bis 3 gelöst ist.

15. Stabile Formulierung nach einem der Ansprüche 1 bis 13 oder hergestellt nach dem Verfahren gemäß Anspruch 14 für die Verwendung als Medikament, vorzugsweise für die Verwendung als Medikament für die Behandlung von einem diabetischen Zustand.

## Revendications

1. Formulation stable pour injection parentérale comprenant :
(a) un peptide ou un de ses sels qui a été préalablement déshydraté à partir d'une composition aqueuse comprenant un acide fort, où le peptide déshydraté ou un de ses sels a un premier profil d'ionisation qui correspond à la stabilité et la solubilité optimales du peptide ; et
(b) un solvant polaire aprotique, où le peptide déshydraté ou un de ses sels est reconstitué dans un solvant polaire aprotique et a un second profil d'ionisation dans le solvant polaire aprotique,
où les premier et second profils d'ionisation se situent dans 1 unité de pH l'un de l'autre,
où la composition aqueuse ne comprend pas de tampon non volatil, et
où le peptide ou un de ses sels est le glucagon.

2. Formulation stable selon la revendication 1, dans laquelle le peptide déshydraté est partiellement ou complètement solubilisé dans le solvant polaire aprotique, de préférence complètement solubilisé dans ledit solvant polaire aprotique.

3. Formulation stable selon l'une quelconque des revendications 1 à 2, où la composition aqueuse comprend de l'acide chlorhydrique.

4. Formulation stable selon la revendication 3, où la composition aqueuse ne comprend pas de tampon.

5. Formulation stable selon l'une quelconque des revendications 1 à 4, où la déshydratation est effectuée par lyophilisation, séchage par pulvérisation, dessiccation, congélation de film mince, lyophilisation par pulvérisation, ou une quelconque de leurs combinaisons.

6. Formulation stable selon l'une quelconque des revendications 1 à 5, où la teneur en humidité de la formulation est de moins de 15 %, ou de moins de 10 %, ou de moins de 5 %, ou de moins de 1 %, ou est anhydre.

7. Formulation stable selon l'une quelconque des revendications 1 à 6, dans laquelle le solvant polaire aprotique est choisi parmi le diméthylsulfoxyde (DMSO), la N-méthylpyrrolidone (NMP), l'acétate d'éthyle, le diméthyl-formamide (DMF), le carbonate de propylène, ou leurs mélanges.

8. Formulation stable selon l'une quelconque des revendications 1 à 7, comprenant en outre un co-solvant qui diminue le point de congélation de la formulation, où le co-solvant est choisi parmi l'éthanol, le propylèneglycol, le glycérol, et leurs mélanges.

9. Formulation stable selon l'une quelconque des revendications 1 à 8, comprenant en outre un excipient de stabilisation choisi parmi un sucre, un amidon, et leurs mélanges.

10. Formulation stable selon la revendication 9, dans laquelle l'excipient de stabilisation est le tréhalose.

11. Formulation stable selon l'une quelconque des revendications 9 à 10, dans laquelle l'excipient de stabilisation est présent dans la formulation en une quantité se situant dans la plage allant de 1 % (p/v) à 20 % (p/v).

12. Formulation stable selon l'une quelconque des revendications 1 à 11, dans laquelle le premier ou le second ou les deux profils d'ionisation correspondent au profil d'ionisation du glucagon quand il est solubilisé dans une solution aqueuse ayant une plage de pH de 2 à 3.

13. Formulation stable selon l'une quelconque des revendications 1 à 12, où la formulation stable est comprise dans une seringue, un dispositif d'injection à stylo, un dispositif auto-injecteur, ou un dispositif de pompe.

14. Procédé de fabrication de l'une quelconque des formulations stables selon les revendications 1 à 13 pour injection parentérale, le procédé comprenant :
(a) la déshydratation d'une composition aqueuse comprenant un peptide ou un de ses sels et un acide fort, en une poudre de peptide déshydraté, où la poudre de peptide déshydraté a un premier profil d'ionisation qui correspond à la stabilité et la solubilité optimales du peptide ; et
(b) la reconstitution de la poudre de peptide déshydraté dans un solvant polaire aprotique, où la poudre de peptide déshydraté reconstituée a un second profil d'ionisation dans le solvant polaire aprotique,
où les premier et second profils d'ionisation se situent dans 1 unité de pH l'un de l'autre,
où le peptide ou un de ses sels est le glucagon, et
où la composition aqueuse a une plage de pH de plus de 3 à 7 avant la déshydratation,
et où le premier ou second ou les deux profils d'ionisation correspondent au profil d'ionisation du glucagon quand il est solubilisé dans une solution aqueuse ayant une plage de pH de 2 à 3.

15. Formulation stable selon l'une quelconque des revendications 1 à 13 ou constituée selon la revendication 14 pour utilisation comme médicament, en particulier pour utilisation comme médicament pour le traitement d'un état diabétique.
